(19) Europäisches Patentamt
European Patent Office
Office européen des brevets

(11) Veröffentlichungsnummer: **0 010 058**
**B1**

(12) # EUROPÄISCHE PATENTSCHRIFT

(45) Veröffentlichungstag der Patentschrift: **22.06.83**

(21) Anmeldenummer: **79810076.4**

(22) Anmeldetag: **27.08.79**

(51) Int. Cl.³: **A 01 N 35/10** //C07C131/00, C07C147/00

(54) **Verfahren und Mittel zum Schutz von Pflanzenkulturen vor der phytotoxischen Wirkung starker Herbizide; Oximätherderivate.**

(30) Priorität: **31.08.78 CH 9201/78**

(43) Veröffentlichungstag der Anmeldung:
**16.04.80 Patentblatt 80/8**

(45) Bekanntmachung des Hinweises auf die Patenterteilung:
**22.06.83 Patentblatt 83/25**

(84) Benannte Vertragsstaaten:
**AT BE CH DE FR GB IT NL**

(56) Entgegenhaltungen:
**CH - A - 528 210**
**DE - A - 2 225 190**
**DE - A - 2 639 405**
**DE - A - 2 808 317**
**US - A - 3 503 732**

(73) Patentinhaber: **CIBA-GEIGY AG**
**Patentabteilung Postfach**
**CH-4002 Basel (CH)**

(72) Erfinder: **Föry, Werner, Dr.**
**Benkenstrasse 65**
**CH-4054 Basel (CH)**
Erfinder: **Martin, Henry, Dr.**
**Jupiterstrasse 17**
**CH-4123 Allschwil (CH)**
Erfinder: **Pissiotas, Georg, Dr.**
**Breslauerstrasse 8**
**D-7850 Lörrach (DE)**

**Die Akte enthält technische Angaben, die nach dem Eingang der Anmeldung eingereicht wurden und die nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 010 058 B1

Verfahren und Mittel zum Schutz von Pflanzenkulturen vor der phytotoxischen Wirkung starker Herbizide; Oximätherderivate

Die vorliegende Erfindung betrifft ein Verfahren zum Schutz von Pflanzenkulturen vor der phytotoxischen Wirkung starker Herbizide mittels Oxim-Derivaten sowie einige neue Oxim-Derivate und Mittel, welche meue Oxim-derivate enthalten.

Die als Wirkstoff zu verwendenden Oxim-Derivate entsprechen der Formel I

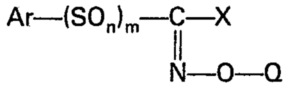

(I)

worin n 0,1 oder 2 und m 0 oder 1 ist, und worin

Ar — einen Phenylrest

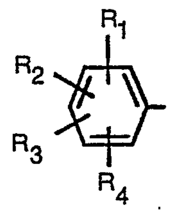

— einen durch $R_2$ und $R_3$ substituierten Naphthylrest,

— einen 5- bis 10-gliedrigen mono- der oder bicyclischen, heterocyclischen Rest, der maximal 3 gleiche oder verschiedene Heteroatome N, O und/oder S enthält und durch $R_2$, $R_3$ und $R_4$ substituiert ist und durch Oxo oder Thiono substituiert sein kann, bedeutet,

$R_1$ — Wasserstoff, Halogen, Niederalkyl, Niederalkoxy oder einen gegebenenfalls maximal zweimal durch Halogen, CN, $NO_2$ oder $CF_3$ substituierten paraständigen Phenoxyrest darstellt,

$R_2$, $R_3$, $R_4$ unabhängig voneinander Wasserstoff, Halogen CN, $NO_2$, Niederalkyl, Niederalkoxy, Halogenalkyl, Halogenalkoxy, Niederalkanoyl, OH, Phenyl, Halogenphenyl, Niederalkylcarbonyloxy, Niederalkoxycarbonyloxy, Nideralkylaminocarbonyloxy, Niederalkylthio, Niederalkylsulfonyl, Phenalkoxy, Cyclohexyl, $NH_2$, -NH-Niederalkyl, -N(Niederalkyl)$_2$, Niederalkanoylamino, Carbonsäureamid oder Sulfonsäureamid bedeuten.

X — für Wasserstoff, —CN, Halogen, Niederalkyl, Niederalkanoyl, —COOH, einen Carbonsäureesterrest oder einen Carbonsäureamidrest steht, und

Q — für den Rest —$C_aH_{2a}$—$R_8$ steht, worin

a eine ganze Zahl von 1 bis 6 bedeutet, wobei der entsprechende Rest auch verzweigt sein kann und $R_8$ für einen der folgenden Rest steht:

—C≡C-Hal —Y—$R_9$ 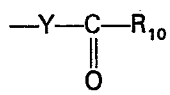 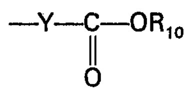

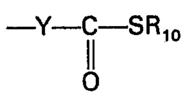 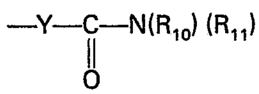 —Y—$SO_2$—$R_{12}$ 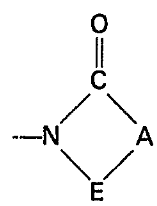

worin

$R_9$ — Wasserstoff, eine gegebenenfalls am Ring ein-oder mehrfach durch CN, $NO_2$, Halogen, Niederalkyl, Niederalkoxy oder Halogenalkyl substituierte cycloaliphatische, araliphatische oder aromatische Gruppe oder Niederalkenyl, Niederhalogenalkenyl oder Niederalkinyl

$R_{10}$ — eine aliphatische Gruppe oder eine gegenbenenfalls am Ring ein- oder mehrfach durch CN, $NO_2$, Halogen, Niederalkyl, Niederalkoxy oder Halogenalkyl substituierte cycloaliphatische, araliphatische oder aromatische Gruppe,

$R_{11}$ — Wasserstoff, Niederalkyl oder Cycloalkyl und

$R_{12}$ — Niederalkyl oder eine aromatische Gruppe bedeuten, während

Y — für O, S, SO, $SO_2$ oder 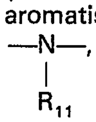

E — für —$CH_2$— oder 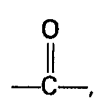

A — für eine gegenbenenfalls durch Halogen, CN oder Niederalkyl substituierte $—CH_2—CH_2—$ oder —CH=CH— Brücke, welche gemeinsam mit dem Fragment

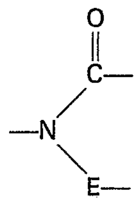

einen 5-gliedrigen Heterocyclus bildet, wobei in Verbindungen, in denen E für

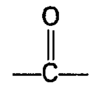

steht, die beiden Kohlenstoffatome dieser Brücke zusammen mit einer gegenbenenfalls durch Halogen, CN oder Niederalkyl substituierten $C_4$-Kette einen ankondensierten, carbocyclischen, 6-gliedrigen Ring bilden können und

Hal — für Halogen stehen.

In der Formel I ist under Halogen Fluor, Chlor, Brom oder Jod zu verstehen.

Carbonsäureester sind Carbonsäureniederalkylester. Carbonsäureamide bedeuten neben $—CONH_2$ auch monoalkylsubstituierte oder symmetrisch oder unsymmetrisch dialkylsubstituierte Amide, wobei die Alkylgruppen Niederalkyl darstellen.

Der Ausdruck Alkyl allein oder als Teil eines anderen Substituierten umfasst verzweigte oder unverzweigte $C_1$- bis $C_8$- Alkylgruppen; Niederalkyl allein oder als Teil eines anderen Substituenten bedeutet $C_1—C_4$ Alkyl. Beispiele sind Methyl, Aethyl, Propyl, Isopropyl, Butyl, Isobutyl, sec.Butyl, tert.Butyl, sowie die höheren Homologen Amyl, Isoamyl, Hexyl, Heptyl, Octyl samt ihren Isomeren. Sinngemäss enthalten Alkanoyle oder Cyanalkyle ein zusätzliches C-Atom. Entsprechend enthalten niedere Alkenyl-oder Alkinylgruppen maximal 4 C-Atome.

Der Begriff aliphatische Gruppe schliesst gesättigte (Alkyle) wie auch ungesättigte (Alkenyle, Alkadienyle, Alkinyle), halogensubstituierte, cyanosubstituierte und durch Sauerstoff unterbrochene Reste eine, die maximal 8 Kohlenstoffatome enthalten.

Der Begriff aromatische Gruppe umfasst Phenyl und Naphthyl, die grundsätzlich ein- oder mehrfach durch CN, $NO_2$, Halogen, Niederalkyl, Niederalkoxy oder Halogenalkyl substituiert sein können. Ein araliphatischer Rest umfasst ein gegebenenfalls ein- bis dreifach substituiertes Phenyl oder Naphthyl, das über Niederalkyl oder Niederalkenyl an den Rest des Moleküls gebunden ist. Beispiele sind die Grundkörper Benzyl, Phenäthyl und Phenylallyl.

Gegebenenfalls substituierte heterocyclische Reste können mono- oder bicyclisch sein. Als Beispiel seien genannat: Furan, Nitrofuran, Bromfuran, Methylfuran, Thiophen, Chlorthiophen, Pyridin, 2,6-Dichlorpyridin, Pyrimidin, Pyridazin, Pyrazin, Piperidin, Methylpiperidin, Morpholin, Thiomorpholin, Tetrahydrofuran, Oxazol, Pyrazol, Pyrrol, Pyrrolin, Pyrrolidin, Thiazol, 2,3-Dihydro-4-H-pyran, Pyran, Dioxan, 1,4-Oxathi-(2)-in, Benzthiazol, Benzoxazol, Benzimidazol, Chinolin, Benz-1,3-dioxolan. Cycloalkylgruppen sind Cyclopropyl, Cyclobutyl, Cyclopentyl, Cyclohexyl und Cycloheptyl, Cycloaliphatische Reste entsprechen diesen Ringsystemen, können daneben aber noch, je nach Möglichkeit, eine oder mehrere Doppelbindungen enthalten.

Die Verbindungen der Formel I können hergestellt werden, indem man

a) mit Ausnahme der Verbindung, worin Q für —C≡C-Hal steht eine Verbindung der Formel II

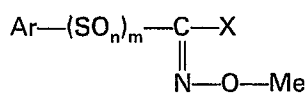

(II)

mit einer Verbindung der Formel III

$$Hal'—Q \qquad (III)$$

umsetzt oder

b) in den Fällen, wo Q für —C≡C-Hal steht, eine Verbindung der Formel IV

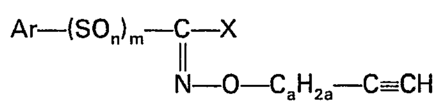

(IV)

mit Halogen in Gegenwart einer Base umsetzt, wobei in den Formeln II, III und IV Ar, X, Q, m und n die

für Formel I gegebenen Bedeutungen haben, Hal' für Halogen, vorzugsweise Chlor oder Brom steht und Me für Wasserstoff oder ein Metallkation, vorzugsweise ein Alkalimetall-oder Erdalkalimetallkation steht.

Die Verbindungen der Formel IV können analog zum Verfahren a) hergestellt werden.

Die Umsetzungen können in An- oder Abwesenheit von gegenüber de Reaktionsteilnehmern inerten Lösungsmitteln durchgeführt werden. Es kommen beispielsweise folgende in Frage: Alkohole wie Aethanol; Ketone wie Aceton; Nitrile wie Acetonitril; N,N-dialkylierte Amide wie Dimethylformamid; Dimethylsulfoxid, Pyridine oder beim Verfahren b) auch Wasser sowie Gemische solcher Lösungsmittel untereinander.

In den Fällen, wo Me für Wasserstoff steht, wird das Verfahren in Gegenwart einer Base durchgeführt, Beispiele geeigneter Basen sind anorganische Basen wie die Oxide Hydroxide, Hydride, Carbonate und Hydrogencarbonate von Alkali und Erdalkalimetallen, sowie z.B. tertiäre Amine wie Trialkylamine (z.B. Triäthylamin), Pyridin usw.

Die Reaktionstemperaturen liegen zwischen 0—150°C vorzugsweise 10—80°C.

Die Reaktionen werden bei Normaldruck und bei a) gegebenenfalls unter einer Stickstoffatmosphäre durchgeführt.

Die Verbindungen der Formel II werden analog an sich bekannter Methoden hergestellt.

Neben der erfindungsgemässen Verwendung von Verbindungen der Formel I zum Schutz von Pflanzenkulturen von der phytotoxischen Wirkung starker Herbizide betrifft die vorliegende Erfindung auch Mittel, welche ein Oxim-Derivat der Formel (I')

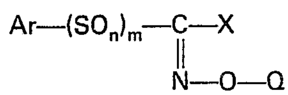

(I')

worin n 0, 1 oder 2 und m 0 oder 1 ist, und worin

Ar — einen Phenylrest

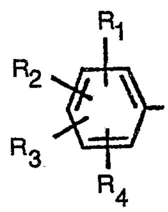

— einen durch $R_2$ und $R_3$ substituierten Naphthylrest,

— einen 5- bis 10-gliedrigen, mono- oder bicyclischen, heterocyclischen Rest, der maximal 3 gleiche oder verschiedene Heteroatome N, O und/oder S enthält und durch $R_2$, $R_3$ und $R_4$ substituiert ist und durch Oxo der Thiono substituiert sein kann, bedeutet,

$R_1$ — Wasserstoff, Halogen, Niederalkyl, Niederalkoxy oder einen gegebenenfalls maximal zweimal durch Halogen, CN, $NO_2$, $CF_3$ substituierten paraständigen Phenoxyrest darstellt,

$R_2$, $R_3$, $R_4$ unabhängig voneinander Wasserstoff, Halogen, CN, $NO_2$, Niederalkyl, Niederalkoxy, Halogenalkyl, Halogenalkoxy, Niederalkanoyl, OH, Phenyl, Halogenphenyl, Niederalkylcarbonyloxy, Niederalkoxycarbonyloxy, Niederalkylaminocarbonyloxy, Niederalkylthio, Niederalkylsulfonyl, Phenalkoxy, Cyclohexyl, $NH_2$, —NH-Niederalkyl, —N(Niederalkyl)$_2$, Niederalkanoylamino, Carbonsäureamid, Sulfonsäureamid bedeuten,

X — für Wasserstoff, CN, Halogen, Niederalkyl, Niederalkanoyl, —COOH, einen Carbonsäureesterrest, einen Carbonsäureamidrest steht, und

Q — für einen Rest —$C_aH_{2a}$—$R_8$ steht,

worin

a eine ganze Zahl von 1 Bis 6 bedeutet, wobei der entsprechende Rest auch verzweigt sein kann und $R_8$ für einen der folgenden Reste steht:

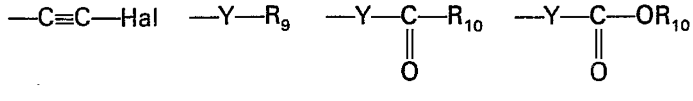

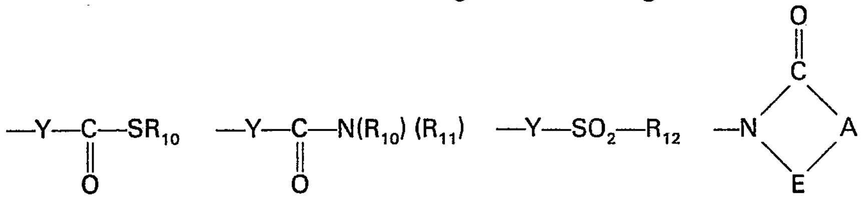

worin

$R_9$ — Wasserstoff, eine gegebenenfalls am Ring ein- oder mehrfach durch CN, $NO_2$, Halogen, Niederalkyl, Niederalkoxy oder Halogenalkyl substituierte cycloaliphatische, araliphatische oder aromatische Gruppe oder Niederalkenyl, Niederhalogenalkenyl oder Niederalkinyl,

$R_{10}$ — eine aliphatische Gruppe oder eine gegebenenfalls am Ring ein- oder mehrfach durch CN, $NO_2$, Halogen, Niederalkyl, Niederalkoxy oder Halogenalkyl substituierte cycloaliphatische, araliphatische oder aromatische Gruppe,

$R_{11}$ — Wasserstoff, Niederalkyl oder Cycloalkyl und

$R_{12}$ — Niederalkyl oder eine aromatische Gruppe bedeuten, während

Y — für O, S, SO, $SO_2$ oder 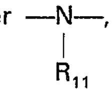,

E — für $—CH_2—$ oder 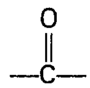

A — für eine gegebenenfalls durch Halogen, CN oder Niederalkyl substituierte $—CH_2—CH_2—$ oder —CH=CH— Brücke, welche gemeinsam mit dem Fragment

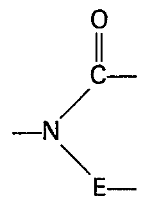

einen 5-gliedrigen Heterocyclus bildet, wobei in Verbindungen, in denen E für

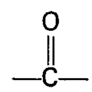

steht, die beiden Kohlenstoffatome dieser Brücke zusammen mit einer gegebenenfalls durch Halogen, CN oder Niederalkyl substituierten $C_4$-Kette einen ankondensierten, carbocyclischen, 6-gliedrigen Ring bilden können, und

Hal — für Halogen

stehen, enthält, mit der Massgabe, dass

i) in Verbindung, in denen $R_8$ für den Rest —C≡C—Hal steht, X nicht für Wasserstoff, —CN oder Niederalkyl steht;

ii) in Verbindungen, in denen $R_8$ für eine Amino- oder Methylaminogruppe oder eine Tosyloxygruppe steht, X nicht für Wasserstoff oder Niederalkyl steht; oder

iii) in Verbindungen, in denen Ar für einen 4-Chlor- oder 4-Bromphenylrest und X für Methyl steht, Q nicht für 2-Hydroxyäthyl, 2-(Alkylcarbonyloxy)-äthyl oder 2-(Alkoxycarbonyloxy)-äthyl steht.

Die Verbindungen der Formel I können für sich allein oder zusammen mit geeigneten Trägern und oder anderen Zuschlagstoffen verwendet werden. Geeignete Träger und Zuschlagstoffe können fest oder flüssig sein und entsprechen den in der Formulierungstechnik üblichen Stoffen wie z.B. natürlichen oder regenerierten mineralischen Stoffen, Lösungs-, Dispergier-, Netz-, Haft-, Verdickungs-, Binde- oder Düngemitteln.

Der Gehalt an Wirkstoff in handelsfähigen Mitteln liegt zwischen 0,1 bis 90%.

Zur Applikation können die Verbindungen der Formel I in den folgenden Aufarbeitungsformen vorliegen (wobei die Gewichts-Prozentangaben in Klammern vorteilhafte Mengen an Wirkstoff darstellen):

Feste Aufarbeitungsformen: Stäubemittel und Streumittel (bis zu 10%) Granulate, Umhüllungsgranulate, Imprägnierungsgranulate und Homogengranulate, Pellets (Körner) 1 bis 80%);

Flüssige Aufarbeitungsformen:

a) in Wasser dispergierbare Wirkstoffkonzentrate:

Spritzpulver (wettable powders) und Pasten (25—90% in der Handelspackung, 0,01 bis 15% in gebrauchsfertiger Lösung); Emulsions; und Lösungskonzentrate (10 bis 50%; 0,01 bis 15% in gebrauchsfertiger Lösung);

b) Lösungen (0,1 bis 20%); Aerosole

Als Gegenmittel oder Antidote sind schon verschiedene Stoffe vorgeschlagen worden, welche befähigt sind, die schädigenden Wirkungen eines Herbizids auf die Kulturpflanze spezifisch zu antagonisieren, d.h. die Kulturpflanze zu schützen, ohne dabei die Herbizidwirkung auf die zu bekämpfenden Unkräuter merklich zu beeinflussen.

Dabei kann ein solches Gegenmittel, auch Safener genannt, je nach seinen Eigenschaften zur Vorbehandlung des Saatgutes der Kulturpflanze (Beizung des Samens oder der Stecklinge) oder vor der Saat in die Saatfurchen oder als Tankmischung für sich allein oder zusammen mit dem Herbizid vor oder nach dem Auflaufen der Pflanzen verwendet werden. Vorauflauf-Behandlung schliesst sowohl die

Behandlung der Anbaufläche vor der Aussaat (ppi = "pre plant incorporation") als auch die Behandlung der angesäten, aber noch nicht bewachsenen Anbauflächen ein.

So beschreibt die GB—PS 1'277'557 die Behandlung von Samen bzw. Sprösslingen von Weizen und Sorghum mit gewissen Oxamsäureestern und Amiden vor dem Angriff durch N-Methoxymethyl-2',6'-diäthyl-chloracetanilid (Alachlor). Andere Literaturstellen (DE—OS 1'952'910, DE—OS 2'245'471, FR—PS 2'021'611) schlagen Gegenmittel zur Behandlung von Getreide, Mais- und Reis-Samen zum Schutz gegen den Angriff herbizider Thiolcarbamate vor. In der DE—PS 1'567'075 und der US—PS 3'131'509 werden Hydroxyamino-acetanilide und Hydantoine für den Schutz von Getreidesamen gegenüber Carbamaten wie IPC, CIPC etc. vorgeschlagen. IPC steht für O-Isopropyl-N-phenylcarbamat; CIPC steht für O-Isopropyl-N-3-chlorphenylcarbamat. In der weiteren Entwicklung haben sich alle diese Präparate jedoch als ungenügend erwiesen.

Ueberraschenderweise besitzen Oxime der Formel I die Eigenschaft, Kulturpflanzen vor dem Angriff starker Herbizide zu schützen, insbesondere vor Herbiziden der verschiedensten Stoffklassen, darunter 1,3,5-Triazinen, 1,2,4-Triazinonen, Phenylharnstoffderivaten, Carbamaten, Thiolcarbamaten, Phenoxyessigsäureestern, Phenoxypropionsäureestern, Halogenacetaniliden, Halogenphenoxyessigsäureestern, subst. Phenoxyphenoxyphenoxy-essigsäureestern und -propionsäureestern, Benzoesäurederivaten usw., sofern diese nicht oder ungenügend kulturentolerant sind.

Ein solches Gegenmittel oder Antidote der Formel I kann je nach Anwendungszweck zur Vorbehandlung des Saatgutes der Kulturpflanze (Beizung des Samens oder der Stecklinge) eingesetzt oder vor oder nach der Saat in den Erdboden gegeben werden oder aber für sich allein oder zusammen mit dem Herbizid vor oder nach dem Auflaufen oder Pflanzen appliziert werden. Die Behandlung der Pflanze oder des Saatguts mit dem Antidote kann daher grundsätzlich unabhängig vom Zeitpunkt der Applikation des Herbizids erfolgen. Sie kann jedoch auch gleichzeitig durchgeführt werde (Tankmischung). Vorauflauf-Behandlung schliesst sowohl die Behandlung der Anbaufläche vor der Aussaat (ppi = "pre plant incorporation") als auch die Behandlung der angesäten, aber noch nicht bewachsenen Anbauflächen ein.

Die Aufwandmengen des Antidotes im Verhältnis zum Herbizid richten sich weigehend nach der Anwendungsart. Sofern eine Feldbehandlung vorgenommen wird, verhalten sich die Mengen von Antidote der Formel I zu Herbizid wie 1:100 bis 5:1, bevorzugt 1:20 bis 1:1. Bei Samenbeizung und ähnlichen gezielten Schutzmassnahmen werden jedoch weit geringere Mengen Antidote im Vergleich mit den z.B. später pro Hektar Anbaufläche verwendeten Herbizidmengen benötigt (z.B. ca. 1:3000 bis 1:1000). In der Regel stehen protektive Massnahmen wie Samenbeizung mit einem Antidote der Formel I und mögliche spätere Feldbehandlung mit Herbiziden nur in losem Zusammenhang. Vorbehandeltes Saat- und Pflanzengut kann später in Landwirtschaft, Gartenbau und Forstwirtschaft mit unterschiedlichen Herbiziden in Berührung kommen.

Die Erfindung bezieht sich daher auch auf kulturpflanzenprotektive Mittel, die als Wirkstoff lediglich ein Antidote der Formel I' zusammen mit üblichen Trägerstoffen enthalten. Solche Mittel können gegebenenfalls zusätzlich mit jenem Herbizid gemischt sein, vor deren Einfluss die Kulturpflanze geschützt werden soll.

Kulturpflanzen seien im Rahmen vorliegender Erfindung alle Pflanzen, die in irgendeiner Form Ertragsstoffe produzieren (Samen, Wurzeln, Stengel, Knollen, Blätter, Blüten, Inhaltsstoffe wie Oele, Zucker, Stärke, Eiweiss etc.) und zu diesem Zweck angebaut und gehegt werden. Zu diesen Pflanzen gehören beispielsweise sämtliche Getreidearten, Mais, Reis, Edelhirse, Soja, Bohnen, Erbsen, Kartoffeln, Gemüse, Baumwolle, Zuckerrüben, Zuckerrohr, Erdnüsse, Tabak, Hopfen, dann jedoch auch Zierpflanzen, Obstbäume sowie Bananen, Kakao- und Naturkautschuk-Gewächse. Diese Aufzählung stellt keine Limitierung dar.

Grundsätzlich lässt sich ein Antidote überall dort einsetzen, wo eine Kulturpflanze vor der Phytotoxizität eines Herbizids geschützt werden soll.

Die Erfindung bezeiht sich auch auf ein Verfahren zum Schutz von Kulturpflanzen vor starken Herbiziden, indem ein als Antidote wirkendes Oximderivat der Formel I wahlweise vor oder nach Applikation des Herbizids oder auch gleichzeitig mit dem Herbizid angewendet wird.

Die Erfindung bezieht sich ferner auf das Vermehrungsgut solcher Kulturpflanzen, das protektiv mit einem Oximderivat der Formel I behandelt wurde. Unter dem Begriff "Vermehrungsgut" sind alle generativen Pflanzenteile zu verstehen, die zur Vermehrung der Kulturpflanze eingesetzt werden können. Dazu zählen Samenkörner (Saatgut in engeren Sinne), Wurzeln, Früchte, Knollen, Rhizome, Stengelteile, Zweige (Stecklinge) und andere Pflanzenteile. Es zählen aber auch angekeimte Pflanzen und Jungpflanzen dazu, die nach der Ankeimung oder dem Auflaufen weiterverpflanzt werden sollen. Solche Jungpflanzen lassen sich durch eine vollständige oder partielle Tauchbehandlung vor dem Weiterverpflanzen gezielt schützen.

Die nachfolgenden Substituententypen oder Kombinationen dieser untereinander werden bevorzugt:

Bei Q:— a) $CH_2—C\equiv CJ$

b) $CH_2—O—$Phenyl (gegebenenfalls substituiert)

c) 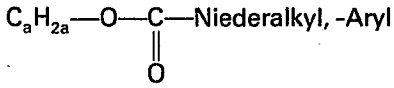

d) 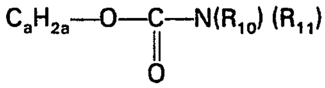

e) 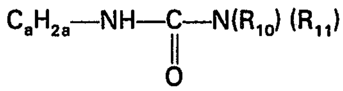

f) $C_aH_{2a}—O—SO_2$-Niederalkyl

g) 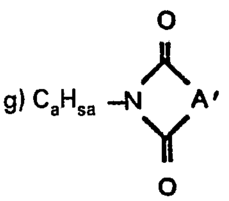 ; A′ = $C_2$-Kette

h) 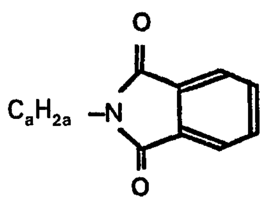

Bei Ar:—

a) 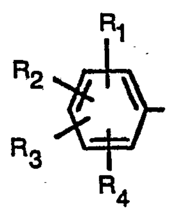

b) 1-Naphthyl
c) Benzoxazol
d) Benzthiazol

Bei X:—
a) Cyano
b) Wasserstoff
c) Ein Carbonsäureesterrest
d) Niederalkyl

Bei n und m:—
a) n = 2 m = 1
b) m = 0

Von besonderem Interesse sind die nachfolgend genannten Einzelverbindungen, die ebenfalls einen Teil der vorliegenden Erfindung darstellen:

Phenyl-acetonitriloxim-succinimidomethyl-äther,
4-Bromphenyl-acetonitriloxim-(3′-jod-2′-propinyl)-äther,
4-Methoxyphenyl-acetonitriloxim-(3′-jod-2′-propinyl)-äther,
Phenyl-acetonitriloxim-phthalimidomethyl-äther,
4-Chlorphenyl-acetonitiloxim-phthalimidomethyl-äther,

2,4-Dichlorphenyl-acetonitriloxim-(2′-vinyloxy-äthyl)-äther,
Phenyl-acetonitriloxim-(2′-vinyloxy-äthyl)-äther,
Phenyl-acetonitriloxim-[2′-(3″-trifluormethylanilido-carbonyloxy)-äthyl]-äther,
4-Methoxyphenyl-acetonitriloxim-benzyloxymethyl-äther,
4-Methylphenyl-acetonitriloxim-benzyloxymethyl-äther,
4-Chlorphenyl-acetonitriloxim-(2′-vinyloxy-äthyl)-äther,
Phenyl-acetonitriloxim-(3′-hydroxy-prop-2′-yl)-äther,
Phenyl-acetonitriloxim-benzylthiomethyl-äther,
4-Chlorphenyl-acetonitriloxim-benzyloxymethyl-äther,
4-Chlorphenyl-acetonitriloxim-(2′-benzyloxy-äthyl)-äther und
Phenyl-acetonitriloxim-(2′-chloracetoxy-äthyl)-äther.

Die nachfolgenden Beispiele dienen zur näheren Erläuterung der Erfindung ohne dieselbe einzuschränken. Die Temperaturen sind in Celsiusgraden angegeben. Prozente und Teile beziehen sich auf Gewicht.

Herstellungsbeispiele

Beispiel 1

Herstellung von

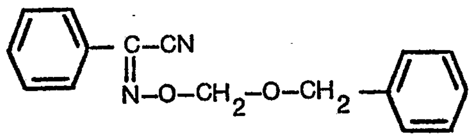

8,4 g (0,05 Mol) des Natriumsalzes von $\alpha$-Phenylacetonitriloxim wurden in 50 ml Dimethylformamid gelöst und mit 7,8 g Chlormethylbenzyläther bei Raumtemperatur unter Rühren versetzt. Nach 8-stündigem Rühren wurde das Reaktionsgemisch mit Wasser gewaschen, mit Essigester extrahiert und die Essigesterlösung über Natriumsulfat getrocknet. Nach Eindampfen im Vakuum wurde das ölige Produkt von $n_D^{22}$ 1,5621 erhalten.

Beispiel 2

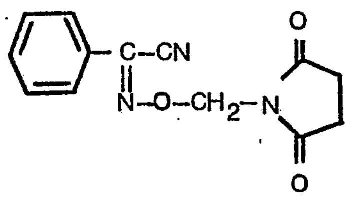

Eine Mischung von 6 g des Natriumsalzes von $\alpha$-Phenylacetonitriloxim und 5,5 g N-Chlormethylsuccinimid in 30 ml Dimethylformamid wurde unter Stickstoffatmosphäre 17 Stunden bei 50° gerührt. Das Reaktionsgemisch wurde mit Essigester verdünnt, mit Wasser gewaschen, getrocknet und eingedampft. Es wurden 9,4 g eines kristallinen Produktes vom Schmelzpunkt 135 bis 138°C erhalten. (Umkristallisiert aus Chloroform/Petroläther).

Beispiel 3

Herstellung von

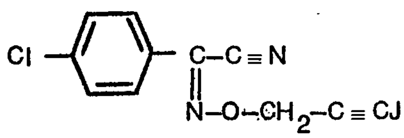

23 g $\alpha$-4-chloracetonitrilpropargyloxim wurden in 150 ml Methanol gelöst. Unter Rühren und Kühlen mit Eiswasser wurden innert 30 Minuten 29,5 g Jod in 100 ml Natronlauge portionenweise bei 5 bis 10°C zugegeben. Nach der Zugabe wurde 4 Stunden bei 5 bis 10° nachgerührt. Danach wurde die Suspension abgenutscht, mit Wasser gewaschen und im Vakuum bei 40 bis 50° getrocknet. Es wurden 33,5 g des Endproduktes vom Schmelzpunkt 84 bis 85° erhalten.

Auf analoge Weise oder nach einer der hierin beschriebenen Methoden können folgende Verbindungen der Formel (I) hergestellt werden.

(Ar = 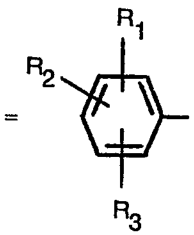 $(SO_2)_m$ )

| Verbindung Nr. | $R_1$ | $R_2$ | $R_3$ | m | X | Q | physikalische Daten |
|---|---|---|---|---|---|---|---|
| 1 | H | H | H | O | CN | $CH_2C \equiv CJ$ | Fp. 83 – 85° |
| 2 | H | 4-Br | H | O | CN | $CH_2C \equiv CJ$ | Oel |
| 3 | 3-Cl | 4-Cl | H | O | CN | $CH_2C \equiv CJ$ | Fp. 77 – 79° |
| 4 | 2-Cl | 4-Cl | H | O | CN | $CH_2C \equiv CJ$ | Fp. 92 – 95° |
| 5 | H | 4-$CH_3O$ | H | O | CN | $CH_2C \equiv CJ$ | Fp. 132 – 134° |
| 6 | H | 4-$CH_3$ | H | O | CN | $CH_2C \equiv CJ$ | Fp. 98 – 100° |
| 7 | 3-$CH_3$ | 4-$CH_3$ | H | O | CN | $CH_2C \equiv CJ$ | Fp. 77 – 79° |
| 8 | H | 4-Cl | H | O | CN | $CH_2C \equiv CJ$ | Fp. 84 – 85° |
| 9 | H | H | H | O | CN | 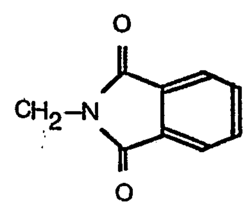 | Fp. 174 – 178 |

| Verbindung Nr. | $R_1$ | $R_2$ | $R_3$ | m | X | Q | physikalische Daten |
|---|---|---|---|---|---|---|---|
| 10 | H | H | H | O | CN | 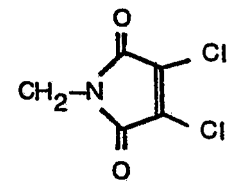 | |
| 11 | H | H | H | O | $COOCH_3$ | 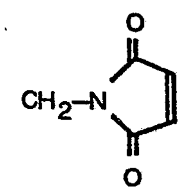 | |
| 12 | H | H | H | O | $COOCH_3$ | 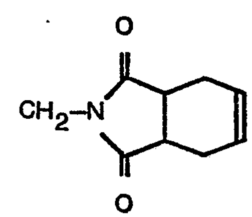 | |
| 13 | H | H | H | O | $CH_3$ | 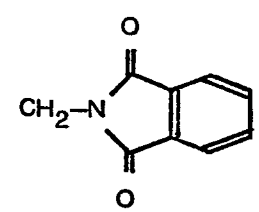 | |

| Verbindung Nr. | $R_1$ | $R_2$ | $R_3$ | m | X | Q | physikalische Daten |
|---|---|---|---|---|---|---|---|
| 14 | H | H | H | O | CN | 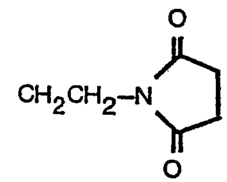 | |
| 15 | H | 4-Cl | H | O | CN | 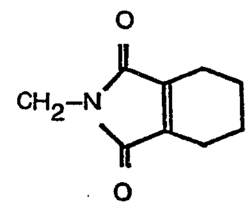 | |
| 16 | 3-Cl | 4-Cl | H | O | CN | 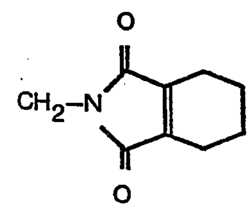 | |
| 17 | H | 4-(t)-$C_4H_9$ | H | O | CN | 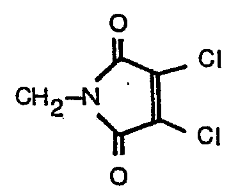 | |

| Verbindung Nr. | $R_1$ | $R_2$ | $R_3$ | m | X | Q | physikalische Daten |
|---|---|---|---|---|---|---|---|
| 18 | H | 4-$CH_3O$ | H | O | CN | 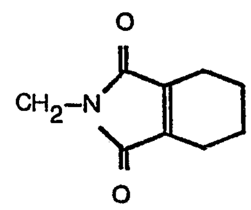 | |
| 19 | 3-$CH_3$ | 4-$CH_3$ | H | O | CN | 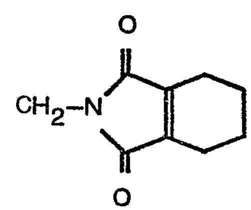 | |
| 20 | H | 4-Cl | H | O | CN | 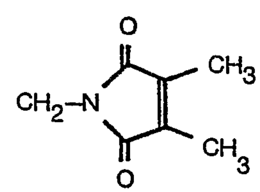 | |
| 21 | 2-Cl | 4-Cl | H | O | CN | $CH_2-O-CH_2-C_6H_5$ | $n_D^{22}$ 1,5745 |
| 22 | H | H | H | O | CN | $CH_2-O-CH_2-C_6H_5$ | $n_D^{22}$ 1,5621 |
| 23 | 2-Cl | 4-Cl | H | O | CN | $CH_2-CH_2-O-CH=CH_2$ | $n_D^{38}$ 1,5510 |

| Verbindung Nr. | $R_1$ | $R_2$ | $R_3$ | m | X | Q | physikalische Daten |
|---|---|---|---|---|---|---|---|
| 24 | H | H | H | 0 | CN | $CH_2-CH_2-O-CH=CH_2$ | $n_D^{25}$ 1,5362 |
| 25 | H | H | H | 1 | CN | $CH_2-C\equiv CJ$ | |
| 26 | H | 4-$CH_3$ | H | 1 | CN | $CH_2-C\equiv CJ$ | |
| 27 | H | 4-Cl | H | 1 | CN | $CH_2-C\equiv CJ$ | |
| 28 | 3-Cl | 4-Cl | H | 1 | CN | $CH_2-C\equiv CJ$ | |
| 29 | H | 4-$CH_3O$ | H | 1 | CN | $CH_2-C\equiv CJ$ | |
| 30 | H | 2-Cl | H | 1 | CN | $CH_2-C\equiv CJ$ | |
| 31 | 2-Cl | 4-Cl | H | 1 | CN | $CH_2-C\equiv CJ$ | |
| 32 | 3-Cl | 4-Cl | H | 1 | $COOCH_3$ | $CH_2-C\equiv CJ$ | |
| 33 | 4-Cl | H | H | 1 | $COOCH_3$ | $CH_2-C\equiv CJ$ | |
| 34 | H | H | H | 1 | $COOCH_3$ | $CH_2-C\equiv CJ$ | |
| 35 | H | H | H | 1 | $COOC_2H_5$ | $CH_2-C\equiv CJ$ | |
| 36 | H | H | H | 0 | CN | $CH_2-CH_2-OH$ | Fp. 56° |
| 37 | 2-Cl | 4-Cl | H | 0 | CN | $CH_2-CH_2-OH$ | $n_D^{26}$ 1,6181 |
| 38 | H | 4-Cl | H | 0 | CN | $CH_2-CH_2-OH$ | Fp. 50° |
| 39 | H | H | H | 0 | CN | $-CH_2CH_2OCONHC_6H_3Cl_2$(3,4) | Fp. 77 – 78° |
| 40 | H | H | H | 0 | CN | $CH_2CH_2OCONHC_6H_4CF_3$(3) | $n_D^{20}$ = 1,5434 |
| 41 | H | H | H | 0 | CN | $CH_2CH_2OCONHC_6H_4Cl$(4) | Fp. 94 – 95° |

| Verbindung Nr. | $R_1$ | $R_2$ | $R_3$ | m | X | Q | physikalische Daten |
|---|---|---|---|---|---|---|---|
| 42 | H | H | H | 0 | CN | $CH_2CH_2OCONHC_6H_5$ | Fp. 71 – 72° |
| 43 | H | H | H | 0 | CN | $CH_2CH_2OCONHCH_3$ | Fp. 57 – 58° |
| 44 | H | H | H | 0 | CN | $CH_2CH_2OCOC_2H_5$ | $n_D^{26}$ = 1,5297 |
| 45 | H | H | H | 0 | CN | $CH_2CH_2OCOCH_2$ | $n_D^{26}$ 1,5322 |
| 46 | H | H | H | 0 | CN | $CH_2CH_2OCOCH_2Cl$ | $n_D^{25}$ 1,5455 |
| 47 | H | H | H | 0 | H | $CH_2CH_2OCOC_6H_5$ | $n_D^{23}$ = 1,5745 |
| 48 | 2-Cl | 4-Cl | H | 0 | H | $CH_2-C\equiv CJ$ | Fp. 92 – 94° |
| 49 | 2-Cl | 6-Cl | H | 0 | H | $CH_2-C\equiv CJ$ | Fp. 83 – 84° |
| 50 | 3-Cl | 4-Cl | H | 0 | H | $CH_2-C\equiv CJ$ | Fp. 83 – 84° |
| 51 | H | 3-$NO_2$ | H | 0 | H | $CH_2-C\equiv CJ$ | Fp. 113 – 114° |
| 52 | H | 4-$NO_2$ | H | 0 | H | $CH_2-C\equiv CJ$ | Fp. 198 – 199° |
| 53 | H | 4-CN | H | 0 | $COOCH_3$ | $CH_2-CH_2-O-C_6H_5$ | |
| 54 | H | 4-CN | H | 0 | $CH_3$ | $CH_2-CH_2-OCONHCH_3$ | |
| 55 | H | 4-CN | H | 0 | CN | $CH_2-CH_2-OCONHCH_3$ | |
| 56 | H | 4-CN | H | 0 | CN | $CH_2-CH_2-OCOCH_3$ | |
| 57 | H | 2-CN | H | 0 | CN | $CH_2-CH_2-OCOCH_3$ | |
| 58 | H | 4-$NO_2$ | H | 0 | $CH_3$ | $CH_2-C\equiv CJ$ | |
| 59 | H | 4-$NO_2$ | H | 0 | CN | $CH_2-C\equiv CJ$ | |

| Verbindung Nr. | $R_1$ | $R_2$ | $R_3$ | m | X | Q | physikalische Daten |
|---|---|---|---|---|---|---|---|
| 60 | H | 2-$CH_2$ | 4-$OCONHCH_3$ | 0 | $CH_3$ | $CH_2-CH_2-O-CONHCH_3$ | |
| 61 | 3-$OCH_3$ | 2-$NO_2$ | 4-$OCOCH_3$ | 0 | CN | 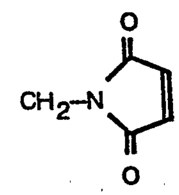 | |
| 62 | 3-Cl | 2-$OCOCH_3$ | 5-Cl | 0 | $CH_3$ | $CH_2-O-C_6H_5$ | |
| 63 | 4-$CH_3$ | 2-$OCOCH_3$ | 6-$CH_3$ | 0 | H | $CH_2CH_2-NHCO-C_6H_5$ | |
| 64 | H | 4-$N(C_2H_5)_2$ | H | 0 | $CH_3$ | $CH_2CH_2-NHCOCH_3$ | |
| 65 | 3-$CH_3$ | 5-$CH_3$ | H | 0 | $CH_3$ | $CH_2CH_2-OCH_2C_6H_3Cl_2(2,4)$ | |
| 66 | H | 4-Cyclohexyl | H | 0 | H | $CH_2CH_2-OH$ | |
| 67 | H | 4-Br | H | 0 | $CONHCH_3$ | $CH_2CH_2-S-CH_2-C_6H_5$ | |
| 68 | H | 2-$OCH_2C_6H_5$ | H | 0 | $CH_3$ | 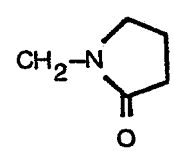 | |

| Verbindung Nr. | $R_1$ | $R_2$ | $R_3$ | m | X | Q | physikalische Daten |
|---|---|---|---|---|---|---|---|
| 69 | H | 4-$C_6H_4Br(4)$ | H | 0 | $CH_3$ | 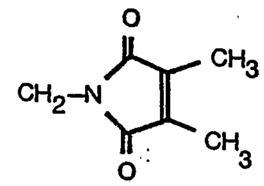 | |
| 70 | H | 4-$OCH_2C_6H_5$ | H | 0 | $CH_3$ | $CH(CH_3)-NHCOCH_3$ | |
| 71 | 3-Cl | 2-$OCOCH_3$ | 6-Cl | 0 | $CH_3$ | $CH_2CH_2-OCOCH_2Cl$ | |
| 72 | H | 4-$CH_3O$ | H | 0 | CN | $CH_2OCH_2C_6H_5$ | $n_D^{24}$ 1,5785 |
| 73 | H | 4-$CH_3$ | H | 0 | CN | $CH_2OCH_2C_6H_5$ | $n_D^{24}$ 1,5655 |
| 74 | H | 4-$CH_3$ | H | 0 | CN | $C_2H_4OCH=CH_2$ | $n_D^{24}$ 1,5159 |
| 75 | H | 4-Cl | H | 0 | CN | $C_2H_4OCH=CH_2$ | Fp. 41 – 43° |
| 76 | H | H | H | 0 | CN | $CH(CH_3)CH_2OH$ | $n_D^{25}$ 1,5492 |
| 77 | H | H | H | 0 | CN | $CH_2SCH_2C_6H_5$ | $n_D^{25}$ 1,5952 |
| 78 | H | 4-Cl | H | 0 | CN | $CH_2OCH_2C_6H_5$ | $n_D^{23}$ 1,5604 |
| 79 | H | 4-Cl | H | 0 | CN | $C_2H_4OCH_2C_6H_5$ | Fp. 74 – 75° |
| 80 | H | H | H | 0 | CN | $-C_2H_4-OC_6H_5$ | Fp. 70 – 71° |
| 81 | H | 4-$CH_3$ | H | 0 | CN | $-C_2H_4-OC_6H_5$ | Fp. 84 – 85° |

| Verbindung Nr. | $R_1$ | $R_2$ | $R_3$ | m | X | Q | physikalische Daten |
|---|---|---|---|---|---|---|---|
| 82 | H | 4-$OCH_3$ | H | 0 | CN | $-C_2H_4-OC_6H_5$ | Fp. 76 – 77° |
| 83 | H | 4-$OCH_3$ | H | 0 | CN | $-C_2H_4-O-CH=CH_2$ | Fp. 57 – 58° |
| 84 | H | 4-$OCH_3$ | H | 0 | CN | $-CH(CH_3)-CH_2-OH$ | $n_D^{24}$ = 1,5379 |
| 85 | H | H | H | 1 | CN | 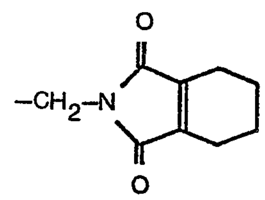 | $n_D^{26}$ : 1,5498 |
| 86 | H | H | H | 1 | CN | 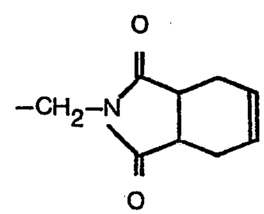 | $n_D^{26}$ : 1,5452 |
| 87 | H | H | H | 1 | CN | 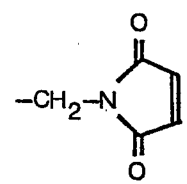 | $n_D^{26}$ : 1,5176 |

| Verbindung Nr. | $R_1$ | $R_2$ | $R_3$ | m | X | Q | physikalische Daten |
|---|---|---|---|---|---|---|---|
| 88 | H | H | H | 1 | CN | 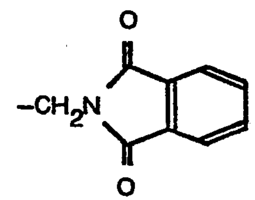 | Fp: 95 – 7°C |
| 89 | H | 4-Cl | H | 1 | CN | 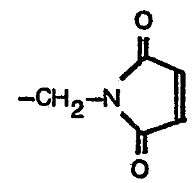 | |
| 90 | H | $4-CH_3$ | H | 1 | CN | 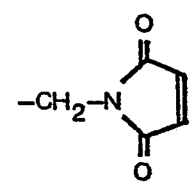 | |
| 91 | H | 4-Cl | H | 1 | CN | 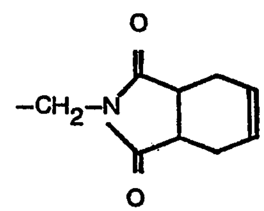 | $n_D^{26}$ = 1,5376 |

| Verbindung Nr. | $R_1$ | $R_2$ | $R_3$ | m | X | Q | physikalische Daten |
|---|---|---|---|---|---|---|---|
| 92 | H | H | 4-Cl | 0 | CN | 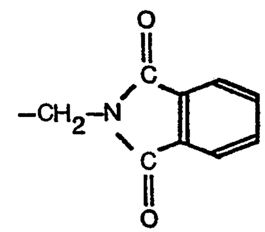 | Fp. 152 – 3° |
| 93 | H | H | H | 0 | CN | 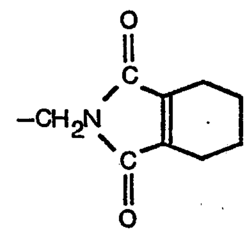 | Fp. 105 – 6° |
| 94 | H | 4-Cl | H | 0 | CN | 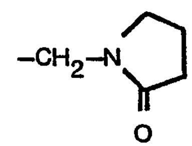 | Fp. 98 – 99° |
| 95 | 2-Cl | 4-Cl | H | 0 | H | $CH_2-C\equiv Cl$ | Fp. 92 – 94° |
| 96 | 2-Cl | H | 6-Cl | 0 | H | $CH_2-C\equiv Cl$ | Fp. 83 – 84° |
| 97 | H | 3-$NO_2$ | H | 0 | H | $CH_2-C\equiv Cl$ | Fp. 113 – 114° |
| 98 | 3-Cl | 4-Cl | H | 0 | H | $CH_2-C\equiv Cl$ | Fp. 83 – 4° |

| Verbindung Nr. | $R_1$ | $R_2$ | $R_3$ | m | X | Q | physikalische Daten |
|---|---|---|---|---|---|---|---|
| 99 | H | 4-$CH_3$ | H | 0 | CN | 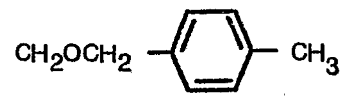 | |
| 100 | H | 4-$OCH_3$ | H | 0 | CN | 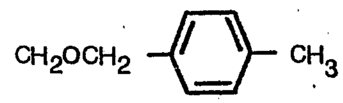 | |
| 101 | 2-Cl | 4-Cl | H | 0 | CN | $CH_2OCH_2C_6H_5$ | |
| 102 | 3-Cl | 4-Cl | H | 0 | CN | $CH_2OCH_2C_6H_5$ | |
| 103 | H | 4-$CH_3$ | H | 0 | CN | 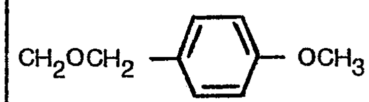 | |
| 104 | H | 4-$CH_3$ | H | 0 | CN | 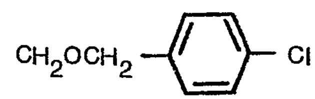 | |
| 105 | 2-$OCH_3$ | H | H | 0 | CN | $CH_2OCH_2C_6H_5$ | |
| 106 | H | 3-$CH_3$ | H | 0 | CN | $CH_2OCH_2C_6H_5$ | |
| 107 | H | 3-Cl | H | 0 | CN | $CH_2OCH_2C_6H_5$ | |
| 108 | H | 3-CN | H | 0 | CN | $CH_2OCH_2C_6H_5$ | |
| 109 | H | 3-$CF_3$ | H | 0 | CN | $CH_2OCH_2C_6H_5$ | |

| Verbindung Nr. | $R_1$ | $R_2$ | $R_3$ | m | X | Q | physikalische Daten |
|---|---|---|---|---|---|---|---|
| 110 | H | 4-Br | H | 0 | CN | $CH_2OCH_2C_6H_5$ | |
| 111 | H | 4-F | H | 0 | CN | $CH_2OCH_2C_6H_5$ | |
| 112 | H | 3-$OCH_3$ | H | 0 | CN | $CH_2OCH_2$ 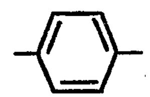 $CH_3$ | |
| 113 | 3-Cl | 4-$CH_3$ | H | 0 | CN | $CH_2OCH_2C_6H_5$ | |
| 114 | H | H | H | 1 | CN | $CH_2OCH_2C_6H_5$ | |
| 115 | H | 4-$CH_3$ | H | 1 | CN | $CH_2OCH_2$ $C_6H_5$ | |
| 116 | H | 4-Cl | H | 1 | CN | $CH_2OCH_2C_6H_5$ | |
| 117 | H | 4-$CH_3$ | H | 0 | $CH_3$ | $CH_2OCH_2C_6H_5$ | |
| 118 | H | 4-$OCH_3$ | H | 0 | $CH_3$ | $CH_2OCH_2C_6H_5$ | |
| 119 | H | 4-Cl | H | 0 | $CH_3$ | $CH_2OCH_2C_6H_5$ | |
| 120 | H | 4-$CH_3$ | H | 0 | H | $CH_2OCH_2C_6H_5$ | |
| 121 | H | 4-$OCH_3$ | H | 0 | H | $CH_2OCH_2C_6H_5$ | |
| 122 | 3-Cl | 4-Cl | H | 0 | H | $CH_2OCH_2C_6H_5$ | |

TABELLE II

(Ar = 1-Naphthyl ; m = O)

| Verbindung Nr. | X | Q | physikalische Daten |
|---|---|---|---|
| 123 | CN | $CH_2CH_2OH$ | $n_D^{26}$ 1,6172 |
| 124 | CN | 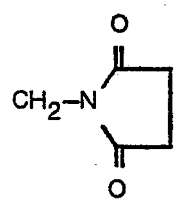 | Fp. 166 – 8° |
| 125 | CN | 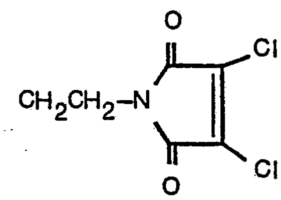 | |
| 126 | CN | $CH_2OCH_2C_6H_5$ | $n_D^{22}$ 1,6300 |
| 127 | CN | $CH_2CH_2OCH=CH_2$ | $n_D^{32}$ 1,5918 |
| 128 | CN | 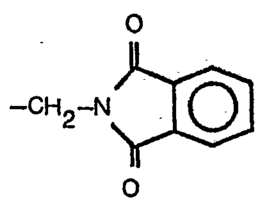 | Smp. 145–146° |

TABELLE III

(Ar = 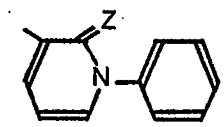 ; m = O) X = H)

| Verbindung Nr. | Z | Q |
|---|---|---|
| 129 | S | $CH_2-CH_2-NH-CO-NH-CH_3$ |
| 130 | S | $CH_2-CH_2-NH-CO-NH-C_6H_4Cl(4)$ |
| 131 | S | $CH_2-CH_2-O-CO-NH-C_6H_3Cl_2(3,4)$ |
| 132 | S | $CH(CH_3)-CH_2-O-C_6H_3-NO_2(2)-CF_3(4)$ |
| 133 | O | $CH_2-C\equiv CJ$ |
| 134 | O | 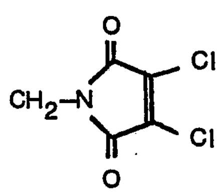 |
| 135 | O | $CH_2-CH_2-O-C_6H_3-NO_2(2)-CF_3(4)$ |

TABELLE IV

(Ar = 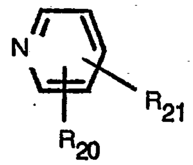 ; m = 0)

| Verbindung Nr. | $R_{20}$ | $R_{21}$ | $-C-X$<br>‖<br>$NO-Q$ |
|---|---|---|---|
| 136 | H | H | 4-$C(CH_3)=NO-CH_2-$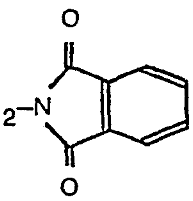 |
| 137 | H | H | 2-$CH=NO-CH_2-O-C_6H_5$ |
| 138 | H | H | 3-$C(CH_3)=NO-CH_2-CH_2-O-C_6H_5Cl_2$(2,4) |
| 139 | H | H | 4-$C(CN)=NO-CH_2-$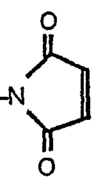 |
| 140 | H | H | 3-$C(CH_3)=NO-CH_2-O-C_6H_5$ |
| 141 | H | H | 3-$C(COOCH_3)=NO-CH_2-O-C_6H_5$ |

TABELLE V

(Ar = 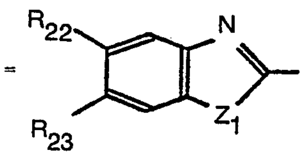 m = O)

| Verbindung Nr. | $Z_1$ | $R_{22}$ | $R_{23}$ | X | Q |
|---|---|---|---|---|---|
| 142 | O | H | H | CN | 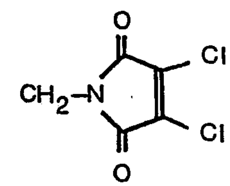 |
| 143 | O | H | H | CN | 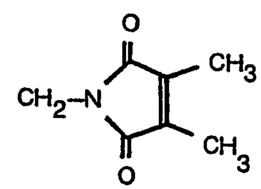 |
| 144 | O | H | H | H | $CH_2-CH_2-O-CO-NH-CH_3$ |
| 145 | O | H | $CH_3$ | CN | 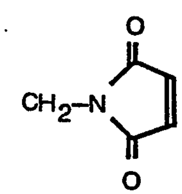 |

TABELLE V (Fortsetzung)

| Verbindung Nr. | $Z_1$ | $R_{22}$ | $R_{23}$ | X | Q |
|---|---|---|---|---|---|
| 146 | O | Cl | H | CN | $CH_2-CH_2-CH_2-O-CON(CH_3)_2$ |
| 147 | S | H | H | CN | $CH_2-CH_2-O-CH_2-C_6H_5$ |
| 148 | S | H | H | Cl | 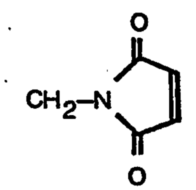 |
| 149 | S | H | H | CN | $CH_2-CH_2-O-CO-NH-CH_3$ |
| 150 | S | H | $CH_3$ | $CH_3$ | $CH_2-CH_2-O-CO-CH_3$ |
| 151 | S | H | $NO_2$ | $CH_3$ | $CH_2-CH_2-OH$ |
| 152 | $N-CH_3$ | H | H | CN | $CH_2-C \equiv CJ$ |
| 153 | $N-CH_3$ | H | H | CN | 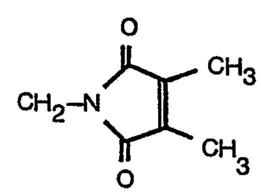 |
| 154 | $N-CH_3$ | H | H | CN | $CH_2-O-C_6H_5$ |

TABELLE V (Fortsetzung)

| Verbindung Nr. | $Z_1$ | $R_{22}$ | $R_{23}$ | X | Q |
|---|---|---|---|---|---|
| 155 | $N-CH_3$ | $CH_3$ | H | CN | 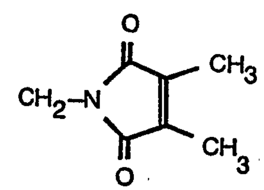 |
| 156 | $N-CH_3$ | Cl | H | $CH_3$ | $CH_2-O-C_6H_5$ |

TABELLE VI

Ar = 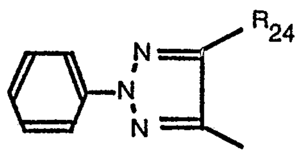 X = H ; m = O)

| Verbindung Nr. | $R_{24}$ | Q |
|---|---|---|
| 157 | H | 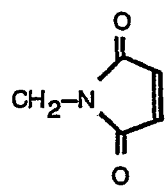 |
| 158 | $CH_3$ | $CH_2-O-C_6H_5$ |
| 159 | H | $CH_2-O-C_6H_5$ |

TABELLE VII

(Ar = 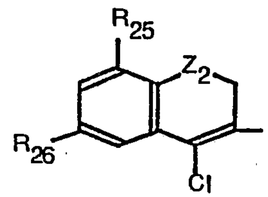 X = H ; m = O)

| Verbindung Nr. | $Z_2$ | $R_{25}$ | $R_{26}$ | Q |
|---|---|---|---|---|
| 160 | O | H | Cl | $CH_2-C\equiv CJ$ |
| 161 | O | H | Cl | $CH_2-CH_2-OH$ |
| 162 | O | Cl | H | $CH_2-CH_2-O-CO-NH-CH_3$ |
| 163 | O | Cl | Cl | $CH_2-C\equiv CJ$ |
| 164 | O | H | H | $CH_2-C\equiv CJ$ |
| 165 | S | H | Cl | 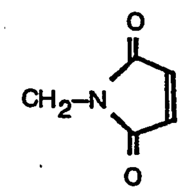 |
| 166 | S | Cl | Cl | $CH_2-CH_2-O-CO-NH-C_6H_4Cl(4)$ |
| 167 | S | H | Cl | $CH_2-CH_2-OH$ |

TABELLE VIII

Ar = ( 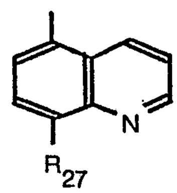 ; X = $CH_3$ ; m = O)

| Verbindung Nr. | $R_{27}$ | Q |
|---|---|---|
| 168 | $O-CO-CH_3$ | $CH_2-O-C_6H_5$ |
| 169 | $O-CO-CH_3$ | $CH_2-O-C_6H_4Cl(4)$ |
| 170 | $O-CO-NH-CH_3$ | $CH_2-O-C_6H_5$ |
| 171 | $O-CO-NH-C_2H_5$ | $CH_2-CH_2-O-C_6H_3-Cl_2(2,4)$ |
| 172 | $O-CO-NH-C_3H_7(i)$ | 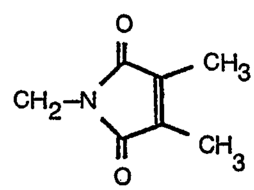 |

## TABELLE IX

(Ar = 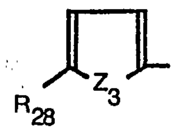 ; m = O)

| Verbindung Nr. | $Z_3$ | $R_{28}$ | X | Q |
|---|---|---|---|---|
| 173 | S | H | CN | $CH_2-O-C_6H_5$ |
| 174 | S | H | Cl | 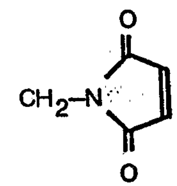 |
| 175 | S | H | CN | $CH_2-CH_2-OH$ |
| 176 | S | H | CN | $CH_2-CH_2-O-CO-NH-CH_3$ |
| 177 | S | Cl | CN | $CH_2-O-C_6H_3-(CH_3)_2-(2,3)$ |
| 178 | S | Cl | $CH_3$ | $CH_2-O-C_6H_4-Cl(4)$ |
| 179 | O | Cl | CN | $CH_2-CH_2-OH$ |
| 180 | O | Cl | $CH_3$ | $CH_2-CH_2-OH$ |
| 181 | O | Cl | Cl | 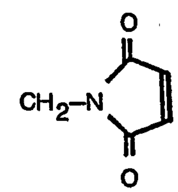 |

TABELLE IX (Fortsetzung)

| Verbindung Nr. | $Z_3$ | $R_{28}$ | X | Q |
|---|---|---|---|---|
| 182 | O | H | CN | $CH_2-CH_2-OH$ |
| 183 | O | H | CN | $CH_2-CH_2-O-CO-NH-CH_3$ |
| 184 | O | H | $CH_3$ | $CH_2-CH_2-OH$ |
| 185 | O | H | $CH_3$ | $CH_2-CH_2-O-CO-NH-C_6H_5$ |
| 186 | O | H | Cl | 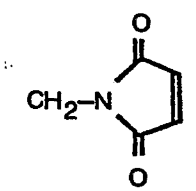 |
| 187 | O | H | CN | $CH_2-C\equiv CJ$ |
| 188 | $N-CH_3$ | H | CN | $CH_2-CH_2-OH$ |
| 189 | $N-CH_3$ | H | CN | $CH_2-CH_2-O-CO-C_2H_5$ |
| 190 | $N-CH_3$ | H | $CH_3$ | $CH_2-C\equiv CJ$ |

Formulierungsbeispiele

Beispiel 4

*Stäubemittel:* Zur Herstellung eines a) 5%igen und b) 2%igen Stäubemittels werden die folgenden Stoffe verwendet:

| | | |
|---|---|---|
| a) | 5 | Teile Wirkstoff |
| | 95 | Teile Talkum; |
| b) | 2 | Teile Wirkstoff |
| | 1 | Teil hochdisperse Kieselsäure |
| | 97 | Teile Talkum; |

Die Wirkstoffe werden mit den Trägerstoffen vermischt und vermahlen und können in dieser Form zur Anwendung verstäubt werden.

Beispiele 5

*Granulat:* Zur Herstellung eines 5%igen Granulates werden die folgenden Stoffe verwendet:

| | |
|---|---|
| 5 | Teile Wirkstoff |
| 0,25 | Teile Epichlorhydrin |
| 0,25 | Teile Cetylpolyglykoläther |
| 3,50 | Teile Polyäthylenglykol |
| 91 | Teile Kaolin (Korngrösse 0,3—0,8 mm). |

Die Aktivsubstanz wird mit Epichlorhydrin vermischt und mit 6 Teilen Aceton gelöst, hierauf wird Polyäthylenglykol und Cetylpolyglykoläther zugesetzt. Die so erhaltene Lösung wird auf Kaolin aufgesprüht, und anschliessend wird das Aceton im Vakuum verdampft.

Beispiel 6

*Spritzpulver:* Zur Herstellung eines a) 70%igen, b) 40%igen c) und d) 25%igen, e) 10%igen Spritzpulvers werden folgende Bestandteile verwendet:

| | | |
|---|---|---|
| a) | 70 | Teile Wirkstoff |
| | 5 | Teile Natriumdibutylnaphthylsulfonat, |
| | 3 | Teile Naphthalinsulfonsäuren-Phenolsulfonsäuren-Formaldehyd-Kondensat 3:2:1, |
| | 10 | Teile Kaolin, |
| | 12 | Teile Champagne-Kreide; |
| b) | 40 | Teile Wirkstoff |
| | 5 | Teile Ligninsulfonsäure-Natriumsalz, |
| | 1 | Teile Dibutylnaphthalinsulfonsäure-Natriumsalz, |
| | 54 | Teile Kieselsäure; |
| c) | 25 | Teile Wirkstoff |
| | 4,5 | Teile Calcium-Ligninsulfonat, |
| | 1,9 | Teile Champagne-Kreide/Hydroxyäthylcellulose-Gemisch (1:1), |
| | 1,5 | Teile Natrium-dibutyl-naphthalinsulfonat, |
| | 19,5 | Teile Kieselsäure, |
| | 19,5 | Teile Champagne-Kreide, |
| | 28,1 | Teile Kaolin; |
| d) | 25 | Teile Wirkstoff |
| | 2,5 | Teile Isooctylphenoxy-polyoxyäthylen-äthanol, |
| | 1,7 | Teile Champagne-Kreide/Hydroxyäthylcellulose-Gemisch (1:1), |
| | 8,3 | Teile Natriumaluminiumsilikat, |
| | 16,5 | Teile Kieselgur, |
| | 46 | Teile Kaolin; |
| e) | 10 | Teile Wirkstoff |
| | 3 | Teile Gemisch der Natriumsalze von gesättigten Fettalkoholsulfaten, |
| | 5 | Teile Naphthalinsulfonsäure/Formaldehyd-Kondensat, |
| | 82 | Teile Kaolin; |

Die Wirkstoffe werden in geeigneten Mischern mit den Zuschlagstoffen innig vermischt und auf entsprechenden Mühlen und Walzen vermahlen. Man erhält Spritzpulver von vorzüglicher Benetzbarkeit und Schwebefähigkeit, die sich mit Wasser zu Suspensionen der gewünschten Konzentration verdünnen und insbesondere zur Behandlung von Pflanzenteilen verwenden lassen.

Beispiel 7

*Emulgierbare Konzentrate:* Zur Herstellung eines 25%igen emulgierbaren Konzentrates werden folgende Stoffe verwendet:

| | |
|---|---|
| 25 | Teile Wirkstoff |
| 2,5 | Teile epoxydiertes Pflanzenöl, |
| 10 | Teile eines Alkylarylsulfonat/Fettalkoholpolyglykoläther-Gemisches, |
| 5 | Teile Dimethylformamid, |
| 57,5 | Teile Xylol. |

Aus solchen Konzentraten können durch Verdünnen mit Wasser Emulsionen der gewünschten Konzentration hergestellt werden, die besonders zur Blattapplikation geeignet sind.

Biologische Beispiele

Beispiel 8

*Vorauflauf-Antidote-Test (Grundtest)*

*Allgemeine Methodik:*

Kleine Blumentöpfe (oberer ϕ 6 cm) werden mit Gartenerde gefüllt, in die die Pflanzenkultur eingesät, bedeckt und leicht festgedrückt wird. Dann wird die als Antidote zu prüfende Substanz als (aus einem Spritzpulver erhaltene) verdünnte Lösung in einer Menge aufgesprüht, die 4 kg AS/ha entspricht. Unmittelbar danach wird in entsprechender Weise das Herbizid aufgesprüht. Nach 18 Tagen Stehen bei ca. 20 bis 23°C und 60 bis 70% relativer Luftfeuchtigkeit wird nach einer linearen Skala von 1 bis 9 ausgewertet, wobei 1 totale Pflanzenschädigung und 9 unbeeinträchtigter Gesundheitszustand bedeuten. Als Kontrolle dienen Pflanzen ohne Antidote-Schutz.

Es wurden verwendet:

1) 1,5 kg As/ha α-[4-(p-Trifluormethylphenoxy)-phenoxy]-propionsäure-n-butoxyäthylester in *Mais* der Sorte "Orla 264".

2) 1,5 kg As/ha Metolachlor = N-(1-Methyl-2-methoxyäthyl)-N-chloracetyl-2-äthyl-6-methylanilin in *Sorghum-Hirse* der Sorte "Funk G—522".

3) 2,0 kg AS/ha Prometryn = 2,4-bis(Isopropylamino)-6-methylthio-s-triazin in *Soja*

4) 2,0 kg AS/ha 4-Aethylamino-6-tert.butylamino-2-chlor-s-triazin in *Weizen* der Sorte "Farnese"

5) 4,0 kg AS/ha Prometryn = 2,4-bis(Isopropylamino)-6-methylthio-s-triazin in *Sorghum-Hirse* der Sorte "Funk G—522".

6) 2,0 kg AS/ha α-[4-(p-Trifluormethylphenoxy)-phenoxy]-propionsäure-n-butoxyäthylester in *Gerste* der Sorte "Mazurka"

In diesen Versuchen wurden z.B. folgende Antidote-Wirkungen erzielt:

| Testvariante | Verbindung Nr. | Note des Herbizideinflusses (ohne/mit Antidote) |
|---|---|---|
| 2 | 2 | 4/6 |
| 2 | 72 | 3/9 |
| 2 | 73 | 3/9 |
| 3 | 5 | 2/6 |
| 4 | 23 | 4/7 |
| 5 | 15 | 3/7 |

Beispiel 9

Antidote-Wirkung bei getrennter Applikation (Antidote-Vorauflauf. Herbizid-Nachauflauf)

*Allgemeine Methodik:*

Kleine Blumentöpfe (oberer ϕ 6 cm) werden mit sandiger Lehmerde gefüllt, in die die Kulturpflanze eingesät wird. Nach dem Bedecken des Samens sprüht man die als Antidote zu prüfende Substanz in verdünnter Lösung und in einer Menge auf die Oberfläche, die umgerechnet 4 kg AS/ha beträgt. Man hält bei 20 bis 23°C und 60 bis 70% relativer Luftfeuchtigkeit. Wenn die Pflanzen nach 10 Tagen das 2- bis 3-Blattstadium erreicht haben, werden sie, wie nachfolgend angegeben, mit der entsprechenden Menge Herbizid behandelt. 14 Tage nach Hebizid-Applikation wird nach einer linearen Skala von 1 bis 9 ausgewertet, wobei 1 totale Pflanzenschädigung und 9 unbeeinträchtigter Gesundheitszustand bedeuten. Als Kontrolle dienen Pflanzen ohne Antidote-Schutz.

Man verwendet:

1) 4,0 kg AS/ha Ametryn = 2-Aethylamino-4-isopropylamino-6-methylthio-s-triazin in *Mais* der Sorte "Orla 264"

2) 1,0 kg AS/ha Prometryn = 2,4-bis (Isopropylamino)-6-methylthio-s-triazin in Sorghum-Hirse der Sorte "Funk G—522"

3) 0,25 kg As/ha $\alpha$-[4-(p-Trifluormethylphenoxy)-phenoxy]-propionsäure-n-butoxyäthylester in *Gerste* der Sorte "Mazurka"

Verbindungen der Formel (I) erzeilten in diesen Versuchen eine gute Antidote-Wirkung. In der Testvariante 2 erreichte die Verbindung No. 80 die Noten 2/6.

Beispiel 10

Antidote-Wirkung an verpflanztem Reis bei getrennter Applikation (Antidote-Vorauflauf. Herbizid-Nachauflauf)

Plastiktöpfe (8 x 8 cm, 10 cm Höhe) werden bis 2 cm unter den Rand mit Erde im sumpfignassen Zustand gefüllt. Die als Antidote zu prüfende Substanz wird in verdünnter Lösung und in einer Menge auf die Oberfläche gesprüht, die 4 kg AS/ha entspricht. Reispflanzen der Sorte "IR—8" werden im 1 1/2- bis 2-Blattstadium in die so vorbereiteten Töpfe verpflanzt. Am nächsten Tag wird der Wasserstand auf ca. 1,5 cm erhöht. 4 Tage nach Verpflanzung werden umgerechnet 0,75 kg AS/ha von 2-Aethylamino-4-(1,2-dimethyl-n-propylamino)-6-methylthio-s-triazin in Granulatform ins Wasser gegeben. Die Temperatur beträgt während der Versuchsdauer 26 bis 28°C, die relative Luftfeuchtigkeit 60 bis 80%. 20 Tage nach Herbizidbehandlung wird nach einer linearen Skala von 1 bis 9 ausgewertet, wobei 1 totale Pflanzenschädigung und 9 unbeeinträchtigter Gesundheitszustand bedeuten. Als Kontrolle dienen Pflanzen ohne Antidote-Schutz.

In diesem Test verringerten die Verbindungen No. 2, 9 und 75 die Note 4 des Herbizideinflusses auf respektive 88, und 7.

Biespiel 11

*Vorauflauf-Antidote-Test in Nährlösung*

Es wird eine Hewitt-Nährlösung hergestellt, die die nachstehend angegebene Menge Herbizid sowie 10 ppm des zu prüfenden Antidotes enthält.

Man verwendet Kultursamen, der in der angegebenen Prüfkonzentration von dem eingesetzten Herbizid erwartungsgemäss geschädigt werden sollte und sät ihn in gekörntes Zonolith (= expandiertes Vermikulit), das sich in einem an der Unterseite durchlöcherten Plastik-Blumentopf (oberer $\phi$ 6 cm) befindet. Dieser wird in einen zweiten durchsichtigen Plastik-Blumentopf (oberer $\phi$ 7 cm) gestellt, in dem sich ca. 50 ml der mit Herbizid und Antidote vorbereiteten Nährlösung befinden, die nunmehr kapillar im Füllmaterial des kleineren Topfes aufsteigt und Samen und keimende Pflanze benetzt. Täglich wird der Flüssigkeitsverlust mit reiner Hewitt-Nährlösung auf 50 ml ergänzt. 3 Wochen nach Testbeginn wird nach einer linearen Skala von 1 bis 9 ausgewertet, wobei 1 totale Pflanzenschädigung und 9 unbeeinträchtigter Gesundheitszustand bedeuten. Die parallel angewandte Kontroll-Lösung enthält keinen Antidote-Zusatz.

Man verwendet:

1) 4 ppm Prometryn = 2,4-bis (Isopropylamino)-6-methylthio-s-triazin in *Sorghum-Hirse* der Sorte "Funk G—522".

2) 4 ppm 4-Aethylamino-6-tert.butylamino-2-chlor-s-triazin in *Weizen* der Sorte "Farnese".

3) 4 ppm $\alpha$-[4-(p-Trifluormethylphenoxy)-phenoxy]-propionsäure-n-butoxyäthylester in *Gerste* der Sorte "Mazurka".

4) 5 ppm Metolachlor = N-(1-Methyl-2-methoxy-äthyl)-N-chloracetyl-2-äthyl-6-methylanilin in *Sorghum-Hirse* der Sorte "Funk G—522".

In diesen Versuchen wurden z.B. folgende Antidote-Wirkungen erzielt:

| Testvariante | Verbindung Nr. | Note des Herbizideinflusses ohne/mit Antidote |
|---|---|---|
| 2 | 23 | 4/7 |
| 2 | 74 | 3/6 |
| 4 | 23 | 4/7 |
| 5 | 78 | 1/6 |

Beispiel 12

*Vorauflauf-Antidote Test in Nährlösung (Reis)*

Es wird eine Hewitt-Nährlösung hergestellt, die zusätzlich 10 ppm des zu prüfenden Antidotes enthält.

Reissamen der Sorte "IR—8" wird in inertes Füllmaterial (gekörntes Zonolith) gesät, des sich in einem an der Unterseite durchlöcherten Plastik-Blumentopf (oberer ϕ 6 cm) befindet. Dieser wird in einen zweiten durchsichtigen Plastik-Blumentopf (oberer ϕ 7 cm) gestellt, in dem sich ca. 50 ml der vorbereiteten Nährlösung befinden, die nunmehr kapillar in Füllmaterial des kleineren Topfes aufsteigt und Samen und keimende Pflanze benetzt. Täglich wird der Flüssigkeitsverlust mit reiner Hewitt-Nährlösung auf 50 ml ergänzt. Nach 15 Tagen werden die Reispflanzen im 2- bis 2 1/2-Blattstadium in rechteckige Plastiktöpfe (8 x 8 cm, 10 cm Höhe) verpflanzt, die mit 500 ml sumpfignasser Erde gefüllt sind. Am nächsten Tag wird der Wasserstand darin auf 1 bis 2 cm über Boden-Niveau erhöht. 4 Tage nach Verpflanzung gibt man das Herbizid 2-Aethylamino-4-(1,2-dimethyl-n-propylamino)-6-methylthio-s-triazin in Granulatform und in einer Menge zu, die umgerechnet 0,75 kg AS/ha beträgt. 3 Wochen nach Herbizidzugabe wird nach einer linearen Skala von 1 bis 9 unbeeinträchtigter Gesundheitszustand bedeuten. Die parallel angewandte Kontroll-Lösung enthält keinen Antidote-Zusatz. In diesem Test erzielten Verbindungen der Formel (I) eine gute Antidote-Wirkung, vor allem die Verbindungen No. 2, 9, 75 and 77.

Beispiel 13

*Nachauflauf-Antidote-Test in Nährlösung*

*Allgemeine Methodik:*

Man füllt kleine Plastik-Blumentöpfe (oberer ϕ 6 cm), die an der Unterseite durchlöchert sind, mit gekörntem Zonolith und sät den Kultursamen ein. Dann wird der Topf in einen zweiten durchsichtigen Plastik-Blumentopf (oberer ϕ 7 cm) gestellt, in dem sich 50 ml Wasser befinden, das kapillar aufsteigt und den Samen benetzt. Ab 5. Tag wird der laufende Wasserverlust mit Hewitt-Nährlösung ausgeglichen. Ab 15. Tag, wenn sich die Kulturpflanze im 1 1/2- bis 2-Blattstadium befindet, wird in die wieder auf 50 ml ergänzte Nährlösung

10 ppm des zu prüfenden Antidotes + die unten angegebene Menge Herbizid

zugegeben. Ab 16. Tag wird der Flüssigkeitsverlust wieder durch reine Hewitt-Nährlösung ausgeglichen. Während der gesamten Testdauer beträgt die Temperatur 20 bis 23°C bei einer relativen Luftfeuchtigkeit von 60 bis 70%. 3 Wochen nach Zugabe des Herbizids und des Antidotes erfolgt die Auswertung nach einer linearen Skala von 1 bis 9, wobei 1 totale Pflanzenschädigung und 9 unbeeinträchtigter Gesundheitszustand bedeuten.

*Testvarianten:*

1) 15 ppm α[4-(3,5-Dichlorpyridyl-2-oxy)-phenoxy]-propionsäure-propargylthioloester in *Weizen* der Sorte "Zenith".

2) 4 ppm 4-Aethylamino-6-tert.butylamino-2-chlor-s-triazin in *Weizen* der Sorte "Zenith".

3) 2 ppm α-[4-(p-Trifluormethylphenoxy)-phenoxy]-propionsäure-n-butoxyäthylester in *Mais* der Sorte "Orla".

4) 8 ppm α-[4-(p-Trifluormethylphenoxy)-phenoxy]-propionsäure-n-butoxyäthylester in *Sorghum-Hirse* der Sorte "Funk G—522".

5) 4 ppm Prometryn = 2,4-bis(Isopropylamino)-6-methylthio-s-triazin in *Sorghum-Hirse* der Sorte "Funk G—522".

6) 8 ppm α[4-(3,5-Dichlorpyridyl-2-oxy)-phenoxy]-propionsäuremethylester in *Weizen* der Sorte "Zenith".

Verbindungen der Formel (I) erzielen bei diesen Versuchen gute Antidote-Wirkung wie z.B. folgendes Resultat:

| Testvariante | Verbindung Nr. | Note des Herbizideinflusses (ohne/mit Antidote) |
|---|---|---|
| 1 | 5 | 4/7 |
| 2 | 23 | 4/7 |
| 2 | 74 | 3/6 |

Beispiel 14

*Antidote Test Samenquellung (Seed Soaking)*

Reissamen der Sorte IR 8 werden während 48 Stunden mit Lösungen der Testsubstanzen von 10, 100 oder 1000 ppm getränkt. Anschliessend werden die Samen etwa 2 Stunden trocknen gelassen, bis sie nicht mehr kleben. Rechteckige Plastiktöpfe (8 x 8 cm, 10 cm Höhe) werden bis 2 cm unter den Rand mit sandigem Lehm gefüllt. 4 g Samen werden pro Topf gesät und nur ganz schwach gedeckt (etwa Durchmesser des Samenkorns). Die Erde wird in einem feuchten (nicht sumpfigen) Zustand gehalten. Dann wird das Herbizid N-(1-Methyl-2-methoxyäthyl)-N-chloracetyl-2-äthyl-6-methylanilin in verdünnter Lösung und in einer Menge appliziert, die umgerechnet 1,5 kg AS/ha entspricht. 7 und 18 Tage nach dem Verpflanzen wird nach einer linearen Skala von 1 bis 9 ausgewertet, wobei 1 totale Pflanzenschädigung und 9 unbeeinträchtigter Gesundheitszustand bedeuten.

In diesem Test erzielen Verbindungen der Formel (I) eine gute Antidote-Wirkung, vor allem die No. 9 (3/6) 76 (3/6) 142 (2/6) 144 (2/6) und 40 (2/6).

**Patentansprüche**

1. Ein Verfahren zum Schutz von Pflanzenkulturen vor der phytotoxischen Wirkung starker Herbizide durch Behandlung mit einem Gegenmittel, dadurch gekennzeichnet, dass man als Gegenmittel ein Oxim-Derivat der Formel (I)

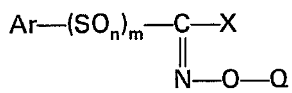

(I)

verwendet,
worin n 0, 1 oder 2 und m 0 oder 1 ist, und worin

Ar — einen Phenylrest

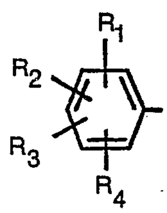

— einen durch $R_2$ und $R_3$ substituierten Naphthylrest,

— einen 5- bis 10-gliedrigen, mono- oder bicyclischen heterocyclischen Rest, der maximal 3 gleiche oder verschiedene Heteroatome N, O und/oder S enthält und durch $R_2$, $R_3$ und $R_4$ substituiert ist und durch Oxo oder Thiono substituiert sein kann, bedeutet,

$R_1$ — Wasserstoff, Halogen, Niederalkyl, Niederalkoxy oder einen gegebenenfalls maximal zweimal durch Halogen, CN, $NO_2$, $CF_3$ substituierten paraständigen Phenoxyrest darstellt,

$R_2$, $R_3$, $R_4$ unabhängig voneinander Wasserstoff, Halogen, CN, $NO_2$, Niederalkyl, Niederalkoxy, Halogenalkyl, Halogenalkoxy, Niederalkanoyl, OH, Phenyl, Halogenphenyl, Niederalkylcarbonyloxy, Niederalkoxycarbonyloxy, Niederalkylaminocarbonyloxy, Niederalkylthio, Niederalkylsulfonyl, Phenalkoxy, Cyclohexyl, $NH_2$, —NH-Niederalkyl, —N(Niederalkyl)$_2$, Niederalkanoylamino, Carbonsäureamid, Sulfonsäureamid bedeuten,

X — für Wasserstoff, —CN, Halogen, Niederalkyl, Niederalkanoyl, —COOH, einen Carbonsäureesterrest, einen Carbonsäureamidrest steht, und

Q — für den Rest $—C_aH_{2a}—R_8$ steht,

worin

a eine ganze Zahl von 1 bis 6 bedeutet, wobei der entsprechende Rest auch verzweigt sein kann und $R_8$ für einen der folgenden Reste steht:

—C≡C—Hal —Y—$R_9$ 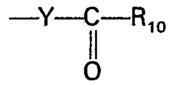 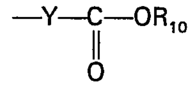

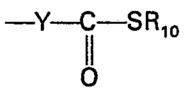 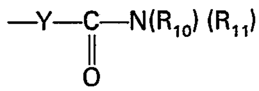 —Y—$SO_2$—$R_{12}$ 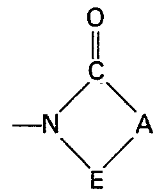

worin

$R_9$ — Wasserstoff, eine gegebenenfalls am Ring ein- oder mehrfach durch CN, $NO_2$, Halogen, Niederalkyl, Niederalkoxy oder Halogenalkyl substituierte cycloaliphatische, araliphatische oder aromatische Gruppe oder Niederalkenyl, Niederhalogenalkenyl oder Niederalkinyl

$R_{10}$ — eine aliphatische Gruppe oder eine gegebenenfalls am Ring ein- oder mehrfach durch CN, $NO_2$, Halogen, Niederalkyl, Niederalkoxy oder Halogenalkyl substituierte cycloaliphatische, araliphatische oder aromatische Gruppe,

$R_{11}$ — Wasserstoff, Niederalkyl oder Cycloalkyl und

$R_{12}$ — Niederalkyl oder eine aromatische Gruppe bedeuten, während

Y — für O, S, SO, $SO_2$ oder 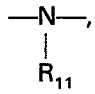,

E für —$CH_2$— oder 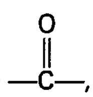,

A — für eine gegebenenfalls durch Halogen, CN oder Niederalkyl substituierte —$CH_2$—$CH_2$— oder —CH=CH— Brücke, welche gemeinsam mit dem Fragment

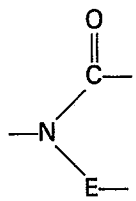

einen 5-gliedrigen Heterocyclus bildet, wobei in Verbindungen, in denen E für

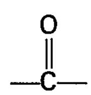

steht, die beiden Kohlenstoffatome dieser Brücke zusammen mit einer gegebenenfalls durch Halogen, CN oder Niederalkyl substituierten $C_4$-Kette einen ankondensierten, carbocyclischen, 6-gliedrigen Ring bilden können und

Hal — für Halogen stehen.

2. Mittel, welches ein Oxim-Derivat der Formel (I')

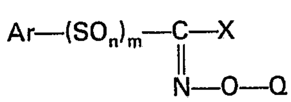 (I'),

worin n 0, 1 oder 2 und m 0 oder 1 ist, und worin

Ar — einen Phenylrest

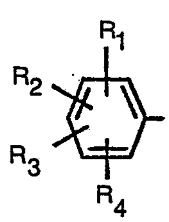

— einen durch $R_2$ und $R_3$ substituierten Naphthylrest,

— einen 5- bis 10-gliedrigen, mono- oder bicyclischen, heterocyclischen Rest, der maximal 3 gleiche oder verschiedene Heteroatome N, O und/oder S enthält und durch $R_2$, $R_3$ und $R_4$ substituiert ist und durch Oxo oder Thiono substituiert sein kann, bedeutet,

$R_1$ — Wasserstoff, Halogen, Niederalkyl, Niederalkoxy oder einen gegebenenfalls maximal zweimal durch Halogen, CN, $NO_2$, $CF_3$ substituierten paraständigen Phenoxyrest darstellt,

$R_2$, $R_3$, $R_4$ unabhängig voneinander Wasserstoff, Halogen, CN, $NO_2$, Niederalkyl, Niederalkoxy, Halogenalkyl, Halogenalkoxy, Niederalkanoyl, OH, Phenyl, Halogenphenyl, Niederalkylcarbonyloxy, Niederalkoxycarbonyloxy, Niederalkylaminocarbonyloxy, Niederalkylthio, Niederalkylsulfonyl, Phenalkoxy, Cyclohexyl, $NH_2$, —NH-Niederalkyl, —N(Niederalkyl)$_2$, Niederalkanoylamino, Carbonsäureamid, Sulfonsäureamid bedeuten,

X— für Wasserstoff, —CN, Halogen, Niederalkyl, Niederalkanoyl, —COOH, einen Carbonsäureesterrest, einen Carbonsäureamidrest steht, und

Q — für einen Rest $—C_aH_{2a}—R_8$ steht,

worin

a eine ganze Zahl von 1 bis 6 bedeutet, wobei der entsprechende Rest auch verzweigt sein kann und $R_8$ für einen der folgenden Reste steht:

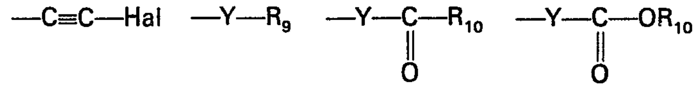

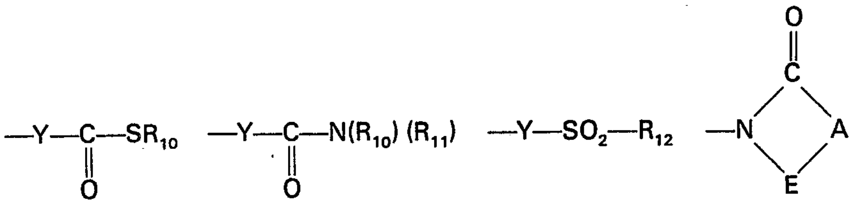

worin

$R_9$ — Wasserstoff, eine gegebenenfalls am Ring ein- oder mehrfach durch CN, $NO_2$, Halogen, Niederalkyl, Niederalkoxy oder Halogenalkyl substituierte cycloaliphatische, araliphatische oder aromatische Gruppe oder Niederalkenyl, Niederhalogenalkenyl oder Niederalkinyl

$R_{10}$ — eine aliphatische Gruppe oder eine gegebenenfalls am Ring ein- oder mehrfach durch CN, $NO_2$, Halogen, Niederalkyl, Niederalkoxy oder Halogenalkyl substituierte cycloaliphatische, araliphatische oder aromatische Gruppe,

$R_{11}$ — Wasserstoff, Niederalkyl oder Cycloalkyl und

$R_{12}$ — Niederalkyl oder eine aromatische Gruppe bedeuten, während

Y — für O, S, SO, $SO_2$ oder 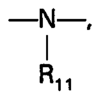,

E — für $—CH_2—$ oder 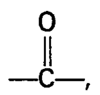,

A — für eine gegebenenfalls durch Halogen, CN oder Niederalkyl substituierte $—CH_2—CH_2—$ oder —CH=CH— Brücke, welche gemeinsam mit dem Fragment

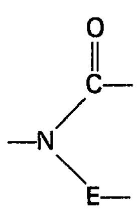

einen 5-gliedrigen Heterocyclus bildet, wobei in Verbindungen, in denen E für

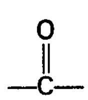

steht, die beiden Kohlenstoffatome dieser Brücke zusammen mit einer gegebenenfalls durch Halogen, CN oder Niederalkyl substituierten $C_4$-Kette einen ankondensierten, carbocyclischen 6-gliedrigen Ring bilden können, und

Hal — für Halogen stehen,

enthält, mit der Massgabe, dass

i) in Verbindung, in denen $R_8$ für den Rest —C≡C—Hal steht, X nicht für Wasserstoff, —CN oder Niederalkyl steht;

ii) in Verbindung, in denen $R_8$ für eine Amino- oder Methylaminogruppe oder eine Tosyloxygruppe steht, X nicht für Wasserstoff oder Niederalkyl steht; oder

iii) in Verbindungen, in denen Ar für einen 4-Chlor- oder 4-Bromphenylrest und X für Methyl steht, Q nicht für 2-Hydroxyäthyl, 2-(Alkylcarbonyloxy)-äthyl oder 2-(Alkoxycarbonyloxy)-äthyl steht.

3. Phenyl-acetonitriloxim-succinimidomethyl-äther.
4. Phenyl-acetonitriloxim-phthalimidomethyl-äther.
5. 4-Chlorphenyl-acetonitriloxim-phthalimidomethyl-äther.
6. 2,4-Dichlorphenyl-acetonitriloxim-(2′-vinyloxy-äthyl)-äther.
7. Phenyl-acetonitriloxim-(2′-vinyloxy-äthyl)-äther.
8. Phenyl-acetonitriloxim-[2′-(3″-trifluormethylanilidocarbonyloxy)-äther]-äther.
9. 4-Methoxyphenyl-acetonitriloxim-benzyloxymethyl-äther.
10. 4-Methylphenyl-acetonitriloxim-benzyloxymethyl-äther.
11. 4-Chlorphenyl-acetonitriloxim-(2′-vinyloxy-äthyl)-äther.
12. Phenyl-acetonitriloxim-(3′-hydroxy-prop-2′-yl)-äther.
13. Phenyl-acetonitriloxim-benzylthiomethyl-äther.
14. 4-Chlorphenyl-acetonitriloxim-benzyloxymethyl-äther.
15. 4-Chlorphenyl-acetonitriloxim-(2′-benzyloxyäthyl)-äther.
16. Phenyl-acetonitriloxim-(2′-chloracetoxy-äthyl)-äther.

## Claims

1. A method of protecting plant crops from the phytotoxic action of potent herbicides by treatment with an antidote, which process comprises using as antidote an oxime derivative of the formula (I)

$$Ar-(SO_n)_m-\underset{\underset{N-O-Q}{\|}}{C}-X \qquad (I)$$

wherein n is 0, 1 or 2 and m is 0 or 1, and

Ar is a phenyl radical

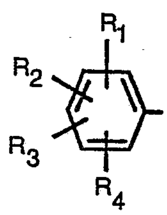

a naphthyl radical substituted by $R_2$ and $R_3$, a 5- to 10-membered mono- or bicyclic heterocyclic radical which contains not more than 3 identical or different heteroatoms N, O and/or S and which is substituted by $R_2$, $R_3$ and $R_4$ and can be substituted by oxo or thiono,

$R_1$ is hydrogen, halogen, lower alkyl, lower alkoxy or a p-phenoxy radical which is unsubstituted or at most disubstituted by halogen, CN, $NO_2$, $CF_3$,

$R_2$ $R_3$ and $R_4$, each independently of the other, are hydrogen, halogen, CN, $NO_2$, lower alkyl, lower alkoxy, haloalkyl, haloalkoxy, lower alkanoyl, OH, phenyl, halophenyl, lower carbalkoxy, lower alkoxycarbonyloxy, lower alkylcarbamoyloxy, lower alkylthio, lower alkylsulfonyl, phenalkoxy, cyclohexyl, $NH_2$, —NH-lower alkyl, —N(di-lower alkyl), lower alkanoylamino, carbamoyl, sulfamoyl,

X is hydrogen, —CN, halogen, lower alkyl, lower alkanoyl, —COOH, a carboxylic acid ester radical, a carbamoyl radical, and

Q is the radical $-C_aH_{2a}-R_8$, wherein a is an integer from 1 to 6, while the corresponding radical can also be branched and $R_8$ is one of the following radicals:

$$-C{\equiv}C-Hal \quad -Y-R_9 \quad -Y-\underset{\underset{O}{\|}}{C}-R_{10} \quad -Y-\underset{\underset{O}{\|}}{C}-OR_{10}$$

$$-Y-\underset{\underset{O}{\|}}{C}-SR_{10} \quad -Y-\underset{\underset{O}{\|}}{C}-N(R_{10})(R_{11}) \quad -Y-SO_2-R_{12}$$

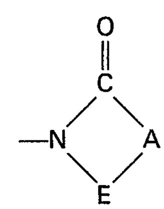

wherein

$R_9$ is hydrogen, a cycloaliphatic, araliphatic or aromatic group which is unsubstituted or mono- or polysubstituted at the ring by CN, $NO_2$, halogen, lower alkyl, lower alkoxy or haloalkyl, or is lower alkenyl, lower haloalkenyl or lower alkynyl,

$R_{10}$ is an aliphatic group or a cycloaliphatic, araliphatic or aromatic group which is unsubstituted or mono- or polysubstituted at the ring by CN, $NO_2$, halogen, lower alkyl, lower alkoxy or haloalkyl,

$R_{11}$ is hydrogen, lower alkyl or cycloalkyl,

$R_{12}$ is lower alkyl or an aromatic group, and

Y is O, S, SO, $SO_2$ or 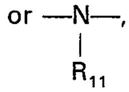,

E is $-CH_2-$ or 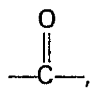,

A is a $-CH_2-CH_2-$ or $-CH{=}CH-$ bridge which is unsubstituted or substituted by halogen, CN or lower alkyl and, together with the fragment

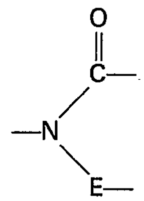

forms a 5-membered heterocyclic ring, whilst in compounds in which E is

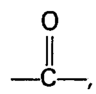,

both carbon atoms of this bridge, together with a $C_4$ chain which is unsubstituted or substituted by halogen, CN or lower alkyl, are able to form a fused carbocyclic 6-membered ring, and

Hal is halogen.

2. A composition which contains an oxime derivative of the formula (I')

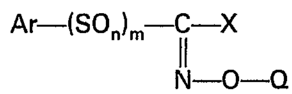

(I'),

wherein n is 0, 1 or 2 and m is 0 or 1, and

Ar is a phenyl radical

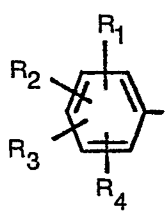

a naphthyl radical substituted by $R_2$ and $R_3$, a 5- to 10-membered mono- or bicyclic heterocyclic radical which contains not more than 3 identical or different heteroatoms N, O and/or S and which is substituted by $R_2$, $R_3$ and $R_4$ and can be substituted by oxo or thiono,

$R_1$ is hydrogen, halogen, lower alkyl, lower alkoxy or a p-phenoxy radical which is unsubstituted or at most disubstituted by halogen, CN, $NO_2$, $CF_3$,

$R_2$, $R_3$ and $R_4$, each independently of the other, are hydrogen, halogen, CN, $NO_2$, lower alkyl, lower alkoxy, haloalkyl, haloalkoxy, lower alkanoyl, OH, phenyl, halophenyl, lower carbalkoxy, lower alkoxycarbonyloxy, lower alkylcarbamoyloxy, lower alkylthio, lower alkylsulfonyl, phenalkoxy, cyclohexyl, $NH_2$, —NH-lower alkyl, —N(di-lower alkyl), lower alkanoylamino, carbamoyl, sulfamoyl,

X is hydrogen, —CN, halogen, lower alkyl, lower alkanoyl, —COOH, a carboxylic acid ester radical, a carbamoyl radical, and

Q is the radical $-C_aH_{2a}-R_8$, wherein a is an integer from 1 to 6, while the corresponding radical can also be branched and $R_8$ is one of the following radicals:

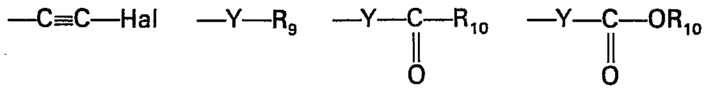

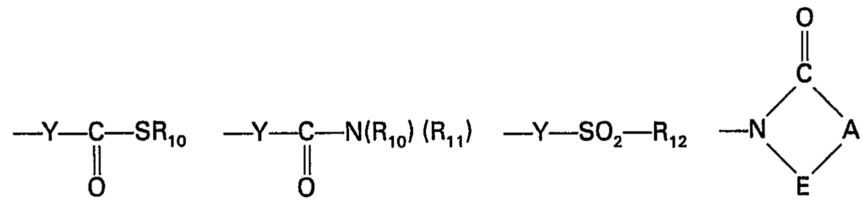

wherein

$R_9$ is hydrogen, a cycloaliphatic, araliphatic or aromatic group which is unsubstituted or mono- or polysubstituted at the ring by CN, $NO_2$, halogen, lower alkyl, lower alkoxy or haloalkyl, or is lower alkenyl, lower haloalkenyl or lower alkynyl,

$R_{10}$ is an aliphatic group or a cycloaliphatic, araliphatic or aromatic group which is unsubstituted or mono- or polysubstituted at the ring by CN, $NO_2$, halogen, lower alkyl, lower alkoxy or haloalkyl,

$R_{11}$ is hydrogen, lower alkyl or cycloalkyl,

$R_{12}$ is lower alkyl or an aromatic group, and

Y is O, S, SO, $SO_2$ or 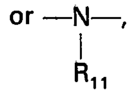,

E is $—CH_2—$ or 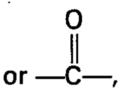,

A is a $—CH_2—CH_2—$ or —CH=CH— bridge which is unsubstituted or substituted by halogen, CN or lower alkyl and, together with the fragment

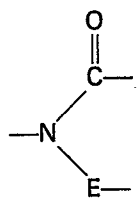

forms a 5-membered heterocyclic ring, whilst in compounds in which E is

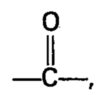,

both carbon atoms of this bridge, together with a $C_4$ chain which is unsubstituted or substituted by halogen, CN or lower alkyl, are able to form a fused carbocyclic 6-membered ring, and

Hal is halogen,

with the proviso that

i) in compounds in which $R_8$ is the radical —C≡C—Hal, X is not hydrogen, —CN or lower alkyl;

ii) in compounds in which $R_8$ is an amino or methylamino group or a tosyloxy group, X is not hydrogen or lower alkyl; or

iii) in compounds in which Ar is a 4-chlorophenyl or 4-bromophenyl radical and X is methyl, Q is not 2-hydroxyethyl, 2-(alkylcarbonyloxy)ethyl or 2-(alkoxycarbonyloxy)ethyl.

3. Phenylacetonitrile-oxime-succinimidomethyl ether.
4. Phenylacetonitrile-oxime-phthalimidomethyl ether.
5. 4-Chlorophenylacetonitrile-oxime-phthalimidomethyl ether.
6. 2,4-Dichlorophenylacetonitrile-oxime-(2′-vinyloxyethyl)ether.
7. Phenylacetonitrile-oxime-(2′-vinyloxyethyl)ether.
8. Phenylacetonitrile-oxime-[2′-(3″-trifluoromethylanilidocarbonyloxy)ethyl]ether.
9. 4-Methyoxyphenyl-acetonitrile-oxime-benzyloxymethyl ether.
10. 4-Methylphenyl-acetonitrile-oxime-benzyloxymethyl ether.
11. 4-Chlorophenylacetonitrile-oxime-(2′-vinyloxyethyl)ether.
12. Phenylacetonitrile-oxime-(3′-hydroxyprop-2′-yl)ether.
13. Phenylacetonitrile-oxime-benzylthiomethyl ether.
14. 4-Chlorophenylacetonitrile-oxime-benzyloxymethyl ether.
15. 4-Chlorophenylacetonitrile-oxime-(2′-benzyloxyethyl)ether.

16. Phenylacetonitrile-oxime-(2′-chloroacetoxyethyl)ether.

**Revendications**

1. Un procédé pour protéger les végétaux cultivés contre l'effet phytotoxique des herbicides forts par traitement à l'aide d'un antidote, caractérisé en ce que l'on utilise en tant qu'antidote un dérivé d'oxime de formule I

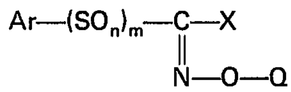

(I)

dans laquelle n est égal à 0, 1 ou 2 et m est égal à 0 ou 1,

Ar représente — un reste phényle

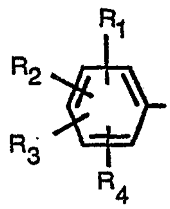

un reste naphtyle substitué par $R_2$ et $R_3$,

un reste hétérocyclique mono- ou bicyclique de 5 à 10 chaînons contenant au maximum 3 hétéroatomes N, O et/ou S indentiques ou différents et qui est substitué par $R_2$, $R_3$ et $R_4$ et peut porter un substituant oxo ou thiono,

$R_1$ représente l'hydrogène, un halogène, un groupe alkyle inférieur, alcoxy inférieur ou un reste phénoxy en position para éventuellement substitué au maximum deux fois par des halogènes, des groupes CN, $NO_2$, $CF_3$,

$R_2$, $R_3$, $R_4$, représentent chacun, indépendamment les uns des autres, l'hydrogène, un halogène, un groupe CN, $NO_2$, alkyle inférieur, alcoxy inférieur, halogénoalkyle, halogénoalcoxy, alcanoyle inférieur, OH, phényle, halogénophényle, (alkyle inférieur)-carbonyloxy, (alcoxy inférieur)-carbonyloxy, (alkylamino inférieur)-carbonyloxy, alkylthio inférieur, alkylsulfonyle inférieur, phénalcoxy, cyclohexyle, $NH_2$, —NH-alkyle inférieur, -N(alkyle inférieur)$_2$, alcanoylamino inférieur, carboxamide, sulfonamide,

X représente l'hydrogène, un groupe —CN, un halogène, un groupe alkyle inférieur, alcanoyle inférieur, —COOH, un reste d'ester d'acide carboxylique, un reste de carboxamide, et

Q représente le reste $-C_aH_{2a}-R_8$ dans lequel

a est un nombre entier de 1 à 6, le reste correspondant pouvant également être ramifié, et

$R_8$ représente l'un des restes suivants:

$-C{\equiv}C-Hal$ $\quad -Y-R_9$ 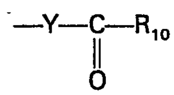 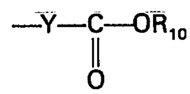

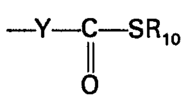 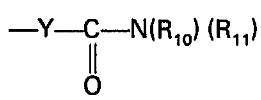 $-Y-SO_2-R_{12}$ 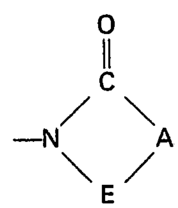

dans lesquels

$R_9$ représente l'hydrogène, un groupe cycloaliphatique, araliphatique ou aromatique éventuellement substitué une ou plusieurs fois dans le noyau par des groupes CN, $NO_2$, des halogènes, des groupes alkyles inférieurs, alcoxy inférieurs, ou halogénoalkyles, ou un groupe alcényle inférieur, halogénoalcényle inférieur ou alcynyle inférieur,

$R_{10}$ représente un groupe aliphatique ou un groupe cycloaliphatique, araliphatique ou aromatique éventuellement substitué une ou plusieurs fois dans le noyau par des groupes CN, $NO_2$, des halogènes, des groupes alkyles inférieurs, alcoxy inférieurs ou halogénoalkyles,

$R_{11}$ représente l'hydrogène, un groupe alkyle inférieur ou cycloalkyle, et

$R_{12}$ représente un groupe alkyle inférieur ou un groupe aromatique, et

Y représente O, S, SO, $SO_2$, ou 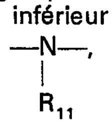,

E représente $-CH_2-$ ou 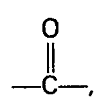,

A représente un pont $—CH_2—CH_2—$ ou $—CH=CH—$ éventuellement substitué par des halogènes, des groupes CN ou alkyles inférieurs et qui forme avec le fragment

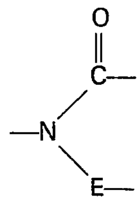

un hétérocycle à 5 chaînons, et en outre, dans les composés dans lesquels E représente

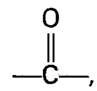

les deux atomes de carbone de ce pont peuvent former avec une chaîne en C 4 éventuellement substituée par des halogènes, des groupes CN ou alkyles inférieurs, un noyau carbocyclique condensé à 6 chaînons et

Hal représente un halogène.

2. Produit contenant un dérivé d'oxime de formule I'

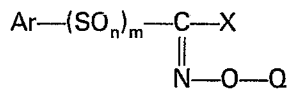

(I'),

dans laquelle n est égal à 0, 1 ou 2 et m à 0 ou 1, et

Ar représente — un reste phényle

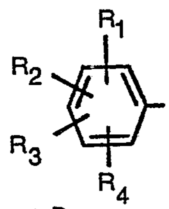

un reste naphtyle substitué par $R_2$ et $R_3$,

un reste hétérocyclique mono- ou bicyclique de 5 à 10 chaînons qui contient au maximum 3 hétéroatomes N, O et/ou S identiques ou différents, est substitué par $R_2$, $R_3$ et $R_4$ et peut porter un substituant oxo ou thiono,

$R_1$ représente l'hydrogène, un halogène, un groupe alkyle inférieur, alcoxy inférieur ou un reste phénoxy en position para éventuellement substitué deux fois au maximum par des halogènes, des groupes CN, $NO_2$, $CF_3$,

$R_2$, $R_3$, $R_4$, représentent chacun, indépendamment les uns des autres, l'hydrogène, un halogène, un groupe CN, $NO_2$, alkyle inférieur, alcoxy inférieur, halogénoalkyle, halogénoalcoxy, alcanoyle inférieur, OH, phényle, halogénophényle, (alkyle inférieur)-carbonyloxy, (alcoxy inférieur)-carbonyloxy, (alkylamino inférieur)-carbonyloxy, alkylthio inférieur, alkylsulfonyle inférieur, phénalcoxy, cyclohexyle, $NH_2$, —NH-alkyle inférieur, —N(alkyle inférieur)$_2$, alcanoyl-amino inférieur, carboxamide, sulfonamide,

X représente l'hydrogène, un groupe —CN, un halogène, un groupe alkyle inférieur, alcanoyle inférieur, —COOH, un reste d'acide carboxylique, un reste de carboxamide, et

Q représente un reste $—C_aH_{2a}—R_8$, dans lequel

a est un nombre entier de 1 à 6, le reste correspondant pouvant également être ramifié, et $R_8$ représente l'un des restes suivants:

$—C\equiv C—Hal$ $—Y—R_9$ 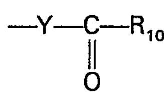 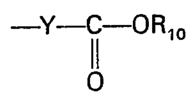

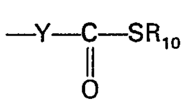 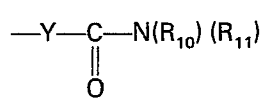 $—Y—SO_2—R_{12}$ 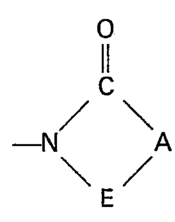

dans lesquels

$R_9$ représente l'hydrogène, un groupe cycloaliphatique, araliphatique ou aromatique éventuellement substitué une ou plusieurs fois dans le noyau par des groupes CN, $NO_2$, des halogènes, des groupes alkyles inférieurs, alcoxy inférieurs ou halogénoalkyles, ou un groupe alcényule inférieur, halogénoalcényle inférieur ou alcynyle inférieur,

$R_{10}$ représente un groupe aliphatique ou un groupe cycloaliphatique, araliphatique ou aromatique éventuellement substitué une ou plusieurs fois dans le noyau par des groupes CN, $NO_2$, des halogènes, des groupes alkyles inférieurs, alcoxy inférieurs ou halogénoalkyles,

$R_{11}$ représente l'hydrogène, un groupe alkyle inférieur ou cycloalkyle, et

$R_{12}$ représente un groupe alkyle inférieur ou un groupe aromatique, et

Y représente O, S, SO, $SO_2$ ou 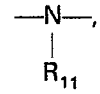

E représente $—CH_2—$ ou 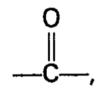

A représente un pont $—CH_2—CH_2—$ ou —CH=CH— éventuellement substitué par des halogènes, des groupes CN ou alkyles inférieurs, qui forme avec le fragment

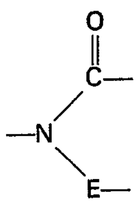

un hétérocycle à 5 chaînons, et en outre dans les composés dans lesquels E représente

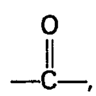

les deux atomes de carbone de ce pont peuvent former avec une chaîne en C 4 éventuellement substituée par des halogènes, des groupes CN ou alkyles inférieurs, un noyau carbocyclique condensé à 6 chaînons, et

Hal représente un halogène, étant spécifié que:

i) dans les composés dans lesquels $R_8$ représente le reste —C≡C—Hal, X ne peut représenter l'hydrogène, un groupe —CN ou alkyle inférieur;

ii) dans les composés dans lesquels $R_8$ représente un groupe amino ou méthylamino ou un groupe tosyloxy, X ne peut représenter l'hydrogène ou un groupe alkyle inférieur; ou bien

iii) dans les composés dans lesquels Ar représente un reste 4-chloro- ou 4-bromo-phényle et X représente un groupe méthyle, Q ne peut représenter un groupe 2-hydroxyéthyle, 2-(alkylcarbonyloxy)-éthyle ou 2-(alcoxycarbonyloxy)-éthyle.

3. L'éther phényl-acétonitriloxime-succinimidométhylique.
4. L'éther phényl-acétonitriloxime-phtalimidométhylique.
5. L'éther 4-chlorophényl-acétonitriloximephtalimidométhylique.
6. L'éther 2,4-dichlorophényl-acétonitriloxime-(2'-vinyloxy-éthylique).
7. L'éther phényl-acétonitriloxime-(2'-vinyloxy-éthylique).
8. L'éther phényl-acétonitriloxime-[2'-(3''-trifluorométhylanilidocarbonyloxy)-éthylique].
9. L'éther 4-méthoxyphényl-acétonitriloxime-benzyloxyméthylique.
10. L'éther 4-méthylphényl-acétonitriloxime-benzyloxyméthylique.
11. L'éther 4-chlorophényl-acétonitriloxime-(2'-vinyloxy-éthylique).
12. L'éther phényl-acétonitriloxime-(3'-hydroxy-propa-2'-ylique).
13. L'éther phényl-acétonitriloxime-benzylthiométhylique.
14. L'éther 4-chlorophényl-acétonitriloxime-benzyloxyméthylique.
15. L'éther 4-chlorophényl-acétonitriloxime-(2'-benzyloxyéthylique).
16. L'éther phényl-acétonitriloxime-(2'-chloracétoxy-éthylique).